(19) Europäisches Patentamt
European Patent Office
Office européen des brevets

(11) Publication number: **0 033 538 B1**

(12) **EUROPEAN PATENT SPECIFICATION**

(45) Date of publication of patent specification: **27.11.85**

(51) Int. Cl.[4]: **C 07 D 309/30,** C 07 C 69/30, A 61 K 31/22, A 61 K 31/365, C 07 C 59/11

(21) Application number: **81100729.3**

(22) Date of filing: **02.02.81**

(54) **6(R)-(2-(8'-acyloxy-2'-methyl-6'-methyl (or hydrogen)-polyhydronaphthyl-1')-ethyl)-4(R)-hydroxy-3,4,5,6-tetrahydro-2H-pyran-2-ones, the hydroxy acid form of said pyranones, the pharmaceutically acceptable salts of said hydroxy acids, and the lower alkyl, and phenyl, dimethylamino or acetylamino substituted lower alkyl esters of said hydroxy acid, processes for preparing the same, and a pharmaceutical antihypercholesterolemic composition containing the same.**

(30) Priority: **04.02.80 US 118049**
**05.08.80 US 175232**
**04.02.80 US 118051**
**05.08.80 US 175460**

(43) Date of publication of application:
**12.08.81 Bulletin 81/32**

(45) Publication of the grant of the patent:
**27.11.85 Bulletin 85/48**

(84) Designated Contracting States:
**AT BE CH DE FR GB IT LI LU NL SE**

(56) References cited:
**EP-A-0 022 478**
**DE-A-3 006 216**
**US-A-3 983 140**
**US-A-4 049 495**
**US-A-4 231 938**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Willard, Alvin K.**
**8 Maplewood Lane**
**Wilmington Delaware 19810 (US)**
Inventor: **Smith, Robert L.**
**1355 Pickwick Lane**
**Lansdale Pennsylvania 19446 (US)**
Inventor: **Hoffman, William F.**
**740 Weikel Road**
**Lansdale Pennsylvania 19446 (US)**

(74) Representative: **Abitz, Walter, Dr.-Ing. et al**
**Abitz, Morf, Gritschneder P.O. Box 86 01 09**
**D-8000 München 86 (DE)**

**EP 0 033 538 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

*SUMMARY OF THE INVENTION*

This invention relates to the group of 6(R) - [2 - (8' - acyloxy - 2' - methyl - 6' - methyl(or hydrogen) - polyhydronaphthyl - 1') - ethyl] - 4(R) - hydroxy - 3,4,5,6 - tetrahydro - 2H - pyran - 2 - ones and to the hydroxy acid form of said pyranones, the pharmaceutically acceptable salts of said hydroxy acids and to the $C_{1-4}$ alkyl and phenyl, dimethylamino, or acetylamino substituted $C_{1-4}$ alkyl esters of said hydroxy acid.

More specifically, this invention relates to a compound of the structure I in Table I, in which the dotted lines X, Y and Z represent possible double bonds, said double bonds being, when any are present, either X and Z together in combination or X, Y or Z alone; R represents $C_{1-10}$ straight or branched chain alkyl (except -2-butyl), $C_{3-10}$ cycloalkyl, $C_{2-10}$ alkenyl, $C_{1-10}$ $CF_3$-substituted alkyl, phenyl, halophenyl, phenyl-$C_{1-3}$ alkyl or substituted phenyl-$C_{1-3}$ alkyl, in which the substituent is halo, $C_{1-3}$ alkyl or $C_{1-3}$ alkoxy; and the free hydroxy acids of formula II formed by opening the lactone ring of formula I in Table I.

The invention also relates to the 6(R) - [2 - (8' - hydroxy - 2',6 - dimethylpolyhydronaphthyl - 1')ethyl] - 4(R) - hydroxy - 3,4,5,6 - tetrahydro - 2H - pyran - 2 - ones as intermediates for the above-described 8'-acyloxy compounds.

*BACKGROUND OF THE INVENTION*

It is known that certain mevalonate derivatives inhibit the biosynthesis of cholesterol, cf. F. M. Singer et al, *Proc. Soc. Exper. Biol. Med., 102,* 370 (1959) and F. H. Hulcher, *Arch. Biochem. Biophys., 146,* 422 (1971). Nevertheless, the activity of these known compounds has not always been found to be satisfactory, i.e. to have practical application.

Recently, Endo et al, reported (U.S. Letters Patent 4,049,495, Patent 4,137,322 and Patent 3,983,140) and DE—A1—3 006 216 which was published after the present priority date) the production of fermentation products which were quite active in the inhibition of cholesterol biosynthesis. The most active member of this group of natural products, now called compactin, IIIa (R'=H) was reported by Brown et al [*J. Chem. Soc. Perkin I* 1165 (1976)] to have a complex mevalonolactone structure.

More recently, Monaghan et al in U.S. Patent 4,231,938, which is incorporated herein by reference, reported an inhibitor, designated MK—803 and having the structure $III_a$ ($R'=CH_3$) in Table I, which was isolated from an entirely different fermentation. Albers-Schonberg et al (EP—A1—0 022 478) described a dihydro MK—803, designated $III_d$ ($R'=CH_3$) in Table I, of equal potency to MK—803 isolated from the same fermentation as was MK—803. Patchett et al (EP—A1—0 033 537) describe dihydro and tetrahydro derivatives of MK—803 of different structures ($III_{b,c\ \text{and}\ e}$ ($R'=CH_3$) in Table I), prepared by the catalytic hydrogenation of MK—803.

A tetrahydro analog $III_e$ (R'=H), of compactin was reported in published Japanese Application (Kokai) 55009—024.

The preparation of the starting materials, $III_a$ and $III_{d'}$ ($R'=CH_3$) are the products of fermentation with a strain of *Aspergillus terreus,* ATCC No. 20542, designated MF—4845 in the culture collection of MERCK & CO., Inc., Rahway, New Jersey, deposited at the American Type Culture Collection, 12301 Parklawn Drive, Rockville, Maryland, USA.

Preparation of Compounds $III_a$ and $III_d$ ($R'=CH_3$)

The following fermentation and isolation steps were carried out in accordance with known processes.

A. *Fermentation*

A tube of lyophilized culture MF—4845 was opened aseptically and the contents suspended in an unbaffled 250 ml Erlenmeyer flask (seed flask) containing approximately 10 ml of the Medium which has the following composition:

*Medium*

| | |
|---|---|
| Corn steep liquor | 5 g |
| Tomato paste | 40 g |
| Oatmeal | 10 g |
| Glucose | 10 g |
| Trace Element Solution | 10 g |
| Distilled water | 1000 ml |
| pH 6.8 with NaOH | |

*Trace Element Solution:*

| | |
|---|---|
| $FeSO_4.7H_2O$ | 1000 mg |
| $MnSO_4.4H_2O$ | 1000 mg |
| $CuCl_2.2H_2O$ | 25 mg |
| $CaCl_2.2H_2O$ | 100 mg |
| $H_3BO_3$ | 56 mg |
| $(NH_4)_6Mo_7O_{24}.4H_2O$ | 19 mg |
| $ZnSO_4.7H_2O$ | 200 mg |
| Distilled Deionized Water | 1000 ml |

The inoculated flask was incubated for 24 hours at 28°C on a 220 rpm shaker (2 inch throw). An unbaffled 2 liter Erlenmeyer flask containing 500 ml of the medium was then inoculated with 10 ml of the first stage fermentation growth from the seed mixture. This too was shaken 24 hours at 28°C.

A 200 gallon stainless steel fermentation vat was then charged with 485 liters of a medium comprising:

| | |
|---|---|
| Cerelose | 4.5% wt/vol |
| Peptonized Milk | 2.5% wt/vol |
| Autolyzed yeast | 0.25% wt/vol |
| Polyglycol P2000 | 0.25% vol/vol |

whose pH was adjusted to 7.0. This was sterilized 15 minutes at 121°C. One liter of the second stage above was then charged and the mixture was incubated at 85 rpm for 12 hours then at 130 rpm for 84 hours at 28°C with an air flow of 5 cfm for 12 hours then 10 cfm for 84 hours.

B. *Isolation*

1. Extraction

Two batches of one hundred gallons of whole broth were combined, acidified with stirring to pH 4.1 by careful addition of 800 ml of concentrated hydrochloric acid, and extracted by addition of 75 gal of ethyl acetate and further stirring for two hours.

About 25 lbs of a silicaceous filter aid was then added and the total slurry was pumped through a 24-inch filter press. An additional 75 gal of ethyl acetate was used to wash the press cake and continue the extraction, by reversing the direction of pumping through the press four times. Then all of the wash solvent was discharged from the press and combined with the first filtrate. The two-phase filtrate was allowed to settle, and the water layer removed. The ethyl acetate layer was washed with 10 gal of deionized water, the phases were allowed to separate and the ethyl acetate extracts were concentrated under vacuum to a residue of about 10 gal.

2. Lactonization

Ethyl acetate extracts from an additional three hundred gal of broth were added to the above extract and the volume was reduced to about thirty gal by vacuum distillation. About fifty gal of toluene was added, and the batch was concentrated under vacuum to 32 gal; this step was repeated; then sufficient new toluene was added to bring the volume to 75 gal. Without vacuum, the batch was brought to reflux and maintained there for two hours, with a temperature over 106°C.

This solution was then concentrated under vacuum to a small volume, which was further concentrated to an oily residue in a large rotary evaporator under vacuum.

3. Chromatography on Silica Gel

The extract obtained above was flushed free of other solvents by addition of 2 gal of methylene chloride and reconcentration to an oil.

The oily residue was dissolved in about 5 gal of ethyl acetate-methylene chloride (30/70; v/v) mixture, and a slurry was made by addition of 2.8 kg of silica gel.

The slurry was loaded as a level layer on the top of a 12 in. × 50 in. silica gel column packed in the same solvent mixture.

Elution was with ethyl acetate-methylene chloride (40/60; v/v) at 800 ml/min. A forerun of 10 gal, then further fractions of 4 gal each were collected.

Fractions 6—10 inclusive were concentrated under vacuum to an oily residue which was dissolved in hot ethyl acetate, treated with decolorizing carbon, filtered hot, and cooled. Crystals of Compound $III_a$ ($R'=CH_3$) were filtered off and the mother liquors were concentrated to an oil for further chromatography. Pure $III_a$ ($R'=CH_3$) has m.p. 170—171°C.

4. Rechromatography on Silica Gel

Mother liquor residues from similar broth extract work-ups equivalent to an additional 600 gal of fermentation production were combined with the above in methylene chloride solution. One-half of this solution was taken for further silica gel chromatography. A small aliquot showed a total solids content of 325 g. The solution was treated with 40 g of decolorizing carbon, filtered, and the cake rinsed with methylene chloride. The combined filtrate and washings were concentrated under vacuum to an oily residue. This was redissolved in 800 ml of ethyl acetate/methylene chloride (30/70; v/v) and slurried with 225 g of silica gel. The slurry was loaded on top of a 14 × 36 cm column bed of silica gel packed in the same solvent mixture. Development was with ethyl acetate/methylene chloride (40/60; v/v). A forecut of three liters was set aside; then fractions of 800 ml each were collected.

5. Chromatography on Reverse-phase Packing

Forty ml from fraction 12 of the above chromatography were concentrated to an oil weighing 500 mg and the oil redissolved in 5 ml acetonitrile. This acetonitrile solution was charged to a $\frac{5}{8}$" OD by 6 ft long stainless steel chromatography column packed with preparative reverse-phase liquid chromatography column packing material "Bondapak C18/PorasilB" (Waters Associates, Inc., Milford, Mass. 01757). The column was eluted with a mixture consisting of (v/v) 55% acetonitrile and 45% 0.05 M ammonium phosphate pH3. The elution volume between 1360 ml and 1700 ml was combined on the basis of refractive index detection. The organic solvent was removed *in vacuo* and the residual aqueous solution extracted with ethyl acetate. *In vacuo* removal of the ethyl acetate left 120 mg of compound which crystallized from a concentrated acetonitrile solution yielding crystals of Compound $III_d$ ($R'=CH_3$), m.p. 129—131°C.

Preparation of Compounds $III_{b,c,e}$

Starting materials $III_b$, $III_c$ and $III_e$ ($R'=CH_3$) are prepared in accordance with the following Flow Sheet and preparative methods.

The desmethyl analogs, $III_b$, $III_c$ and $III_e$ ($R'=H$) are obtained substantially as described by Patchett et al. (European application, filed February 2, 1981, Merck case 16448Y) but starting with $III_a$ ($R'=H$) in each case.

For the preparation of $III_e$ it is advantageous to reduce $III_d$ inasmuch as the desired transfusion of the perhydronaphthalene ring, present in the starting materials, is retained in the final product, and the need to separate isomers is avoided.

FLOW SHEET

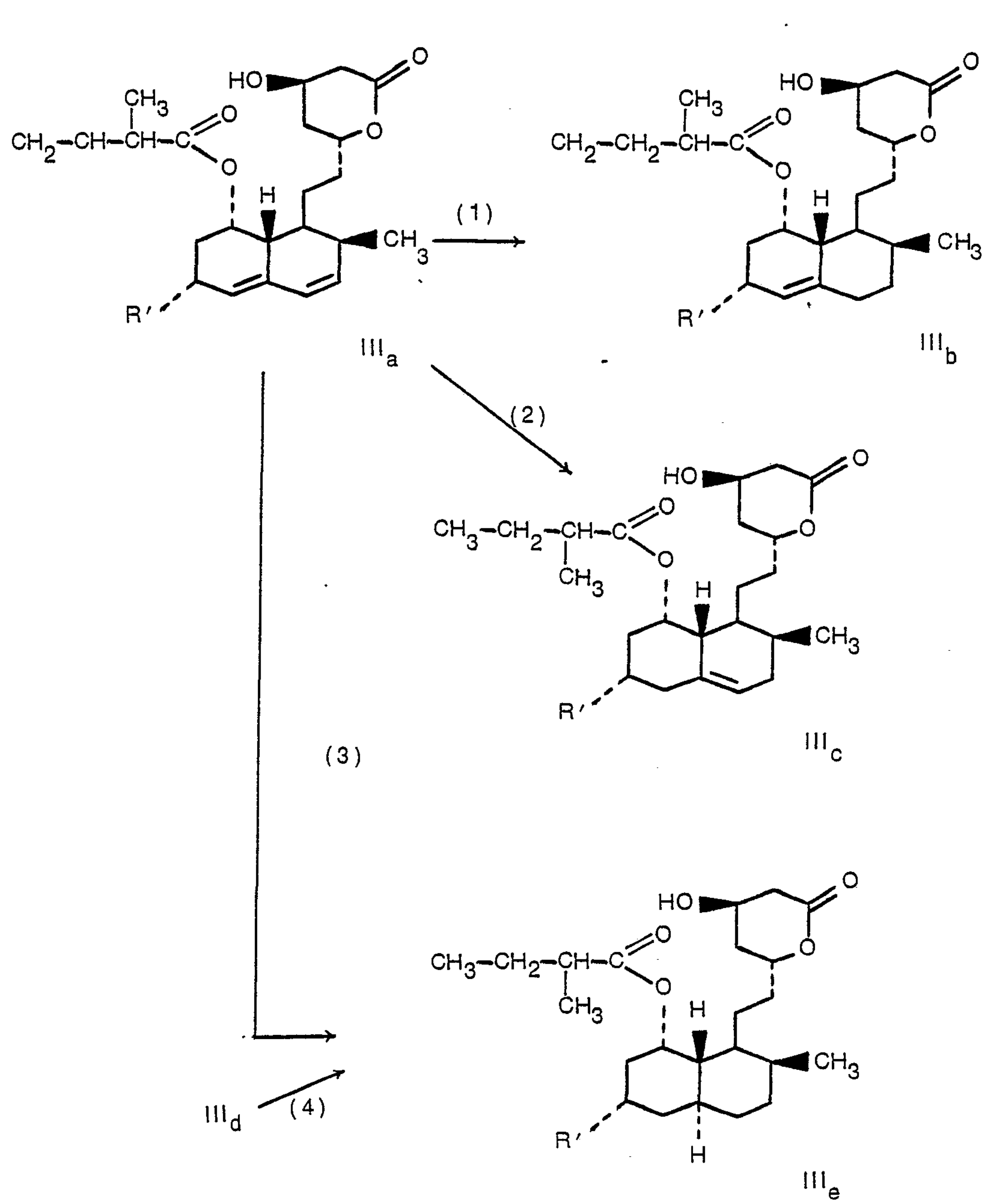


$III_a$ $III_b$ $III_c$ $III_d$ $III_e$

*Reactions and Reagents*

1. Hydrogenation at about 20—75°C and about atmospheric pressure to about 4 atmospheres over tris(triphenylphosphine)chlororhodium in an aromatic solvent such as benzene, toluene or xylene, preferably toluene. Preferred conditions are about 40°C and about 2—7 atmosphers in toluene.
2. Hydrogenation at about 20—25°C and about atmospheric pressure over 5% palladium on calcium carbonate in a lower alkanol such as a $C_{1-3}$ alkanol, especially ethanol.
3. Hydrogenation at about 20—25°C and atmospheric pressure over platinum oxide in ethyl acetate.
4. Hydrogenation at 20—25°C and atmospheric pressure over 10% Palladium on charcoal in ethyl acetate.

Preparation of 6α[2 - (8′β - 2 - (S) - methylbutyryloxy - 2′β,6′α - dimethyl - 1′,2′,3′,4′,6′,7′,8′,8′a - octahydronaphthyl - 1)ethyl] - 4β - hydroxy - 3,4,5,6 - tetrahydro - 2H - pyran - 2 - one, $III_b$ (R′—$CH_3$)

A mixture of 50 mg (0.1236 mmol) of Compound $III_a$ (R′=$CH_3$) and an equal molar amount (114.35 mg, 0.1236 mmol) of tris(triphenylphosphine)chlororhodium in 10 ml of dry toluene was hydrogenated at room temperature for 6 days, with a total uptake of 14.6 ml of hydrogen. The mixture was evaporated *in vacuo* to

dryness. The red residue subjected to preparative thin-layer chromatography on silver nitrate impregnated silica plates and was developed twice in the 10% ethyl acetate-ether system. The yield of Compound $III_b$ ($R'=CH_3$) was 22.3 mg.

Mass spectrum (M/e)
406 ($m^+$)
304 (m−102)
286 (m−102—18)
nmr ($CDCl_3$, 300MHz)
δ 4.37 (m, 1H)
4.60 (m, 1H)
5.34 (d of t, J=2.5 Hz, 1H)
5.41 (m, 1H)

Preparation of 6α[2 - (8′β - 2 - (S) - methylbutyryloxy - 2′β,6′α - dimethyl - 1′,2′,3′,5′,6′,7′,8′,8′a - octahydronaphthyl - 1)ethyl] - 4β - hydroxy - 3,4,5,6 - tetrahydro - 2H - pyran - 2 - one, $III_c$ ($R'$—$CH_3$)

A solution of 80.91 mg (0.2 mmol) of Compound $III_a$ ($R'=CH_3$) in 10 ml of absolute ethanol, in the presence of an equal weight of 5% Pd on $CaCO_3$ was hydrogenated at 1 atmosphere until an uptake of one mole equivalent of hydrogen was observed. The catalyst was then removed by filtration and the filtrate was evaporated to dryness (81 mg). After a purification by preparative thin-layer chromatography to remove a small amount of by-product tetrahydro compound, 72 mg of the 1,4 reduction product $III_c$ ($R'=CH_3$) was isolated.

Mass spectrum (M/e)
406 ($m^+$)
304 (m−102)
286 (304−$H_2O$)
nmr ($CDCl_3$, 300MHz)
δ 4.38 (m, 1H)
4.64 (m, 1H)
5.28 (d of t, J=3.5Hz, 1H)
5.48 (m, 1H)

Preparation of 6α[2 - (8′β - 2(S) - methylbutyryloxy - 2′α,6′β - dimethyl - 1′,2′,3′,4′,4′aα,5′, 6′,7′,8′,8′a - decahydronaphthyl - 1)ethyl] - 4β - hydroxy - 3,4,5,6 - tetrahydro - 2H - pyran - 2 - one, $III_e$ ($R'=CH_3$)

A solution of 80.91 mg (0.2 mmol) of Compound $III_a$ ($R'=CH_3$) in 10 ml of ethyl acetate was hydrogenated in the presence of an equal weight of platinum oxide at one atmosphere. An exact 2 mole equivalent of hydrogen was consumed within 1 hour. The catalyst was removed by filtration and the filtrate was concentrated to dryness to give an oil. The cis and trans isomers were separated by preparative thin-layer chromatography on silica gel plates (10% ethyl acetate-ether system, bands detected by water spray). The trans isomer $III_e$ ($R'=CH_3$) appears as the more polar spot, compared to the cis isomer, and 60 mg was isolated.

Mass spectrum (M/e)
408 (m+)
323 (m−85)
306 (m−102)
nmr ($CDCl_3$, 300MHz)
δ 4.36 (broad singlet, 1H)
4.59 (m, 1H)
5.19 (d of t, J=2.5Hz, 1H)

Fermentative Production of Compound $III_d$ ($R'=H$)

A. *Fermentation:*

A natural isolate of *Penicillium citriunum,* NRRL 8082, deposited at Northern Regional Research Center, Peoria, Illinois, USA, was used to prepare a yeast-malt extract (YME) slant which was incubated for 2 weeks at 28°C.

A portion (1/5) of the slant (MF—4870a) was used to inoculate each of 5 unbaffled seed flasks (250 ml) containing 44 ml of KF seed medium with $CaCl_2$. They were incubated for 3 days at 28°C, and 220 rpm. A portion of the seed growth (about 1.5 ml) was used to inoculate each of 100 production medium flasks (250 ml unbaffled) containing 40 ml of LM Production Medium Without Malt Extract. The production flasks were incubated for 4 days at 25°C.

Another group of production medium flasks (140), each containing 40 ml of LM Production Medium Without Modification were inoculated and incubated under the same conditions as previously described. The broths from both fermentations were combined.

The various media employed in the foregoing fermentations are:

*YME Slant*

| | |
|---|---|
| Dextrose | 4 g./l. |
| Malt Extract | 10 g./l. |
| Yeast Extract | 4 g./l. |
| Agar | 20 g./l. |
| Distilled Water | to 1 liter |
| pH | 7.0 |

*KF Seed Medium with $CaCl_2$*

| | |
|---|---|
| $CaCl_2$ | 10 g. |
| Corn steep liquor | 5 g. |
| Tomatoe Paste | 40 g. |
| Oatmeal | 10 g. |
| Cerelose | 10 g. |
| Trace Element Mix | 10 ml. |
| Distilled Water | 1000 ml. |
| pH | 6.8 |

*Trace Element Mix*

| | |
|---|---|
| $FeSO_4.7H_2O$ | 1 g. |
| $MnSO_4.4H_2O$ | 1 g. |
| $CuCl_2.2H_2O$ | 25 mg. |
| $CaCl_2$ | 100 mg. |
| $H_3BO_3$ | 56 mg. |
| $(NH_4)_6Mo_7O_{24}.4H_2O$ | 19 mg. |
| $ZnSO_4.7H_2O$ | 200 mg. |
| Distilled Water | 1000 ml. |

*LM Production Medium Without Malt Extract*

| | |
|---|---|
| Dextrose | 20 g. |
| Glycerol | 20 ml. |
| Ardamine pH | 10 g. |
| $CoCl_2.6H_2O$ | 8 mg. |
| Polyglycol p 2000 | 0.25% |
| Distilled Water | 1000 ml. |
| pH | 7.0 |

*LM Production Medium Without Modification*

| | |
|---|---|
| Dextrose | 20 g. |
| Glycerol | 20 ml. |
| Ardamine pH | 10 g. |
| Malt Extract | 20 g. |
| $CoCl_2.6H_2O$ | 8 mg. |
| Polyglycol p 2000 | 0.25% |
| Distilled Water | 1000 ml. |
| pH | 7.0 |

B. *Isolation*

The combined whole broth (10.3 liters) was filtered and the mycelia cake was washed with 2.5 liters of deionized water. The combined filtrate and wash was adjusted to pH 4.0 with 1N hydrochloric acid. The aqueous solution was extracted with 7 liters of ethyl acetate and the extract was back-extracted with 3 × 2 liters of aqueous sodium hydroxide solution. The combined sodium hydroxide extract was adjusted to pH 3.8 with 1N hydrochloric acid and extracted with 2 liters and 1 liter of ethyl acetate. The combined ethyl acetate solution was dried over anhydrous $Na_2SO_4$, filtered and concentrated to dryness. The oily residue was dissolved in toluene and refluxed for 1 hour. The toluene solution was concentrated to dryness and the residue was dissolved in 18 ml of a mixture of n-hexane/toluene/methanol (4/1/1 by volume). This solution was loaded onto a 30 mm (ID) × 40 cm. Sephadex LH—20 column equilibrated in the same solvent system. After eluting with 300 ml of solvent, a 10 ml fraction was obtained which was concentrated to an oil. High performance liquid chromatography (HPLC) on an ES Industries Chromega[R] column (9 mm × 50 cm) using a mixture of acetonitrile/water (60/40 by volume) as the eluting solvent yielded 45 mg of dihydrocompactin (Compound $III_d$, R′=H), m.w. 392.2560 by mass spectrum (calculated for $C_{23}H_{36}O_5$, 392.2558).

In KBr, the major IR peaks obtained from a Fourier Transform —IR (FTIR, Nicolet, Model 7199) are at 1724, 1704, 1258, 1078 and 1070 $cm^{-1}$. Of significance is a peak at 3005 $cm^{-1}$ and the absence of a peak at 3030 $cm^{-1}$.

A nuclear magnetic resonance spectrum was obtained in $CDCl_3$, (~1 mg/0.5 ml) on a Varian SC—300 superconducting nmr spectrometer. The following are the peak positions given in ppm (δ) relative to internal tetramethylsilane (TMS).

| δ | Assignment |
|---|---|
| 5.62 d,d,d (2.17, 4.5, 10.0) | $H_{3'}$ (or 4') |
| 5.43 d (10) | $H_{4'}$ (or 3') |
| 5.20 m | $H_{8'}$ |
| 4.63 m | $H_6$ |
| 4.39 m | $H_4$ |
| 2.75 d,d (17.5, 5.5) | |
| 2.63 d,d,d (17.5, 4.0, 1.5) | 3—$CH_2$ |
| 2.39 m | $CH_3\underline{H}C\overset{O}{C}$ |
| 2.29 m | $H_{4a'}+H_{2'}$ |
| 1.14 d | $\underline{CH_3}CH\overset{O}{C}$ |
| 0.90 t | $\underline{CH_3}CH_2$ |
| 0.84 d | 2'—$\underline{CH_3}$ |

d: doublet; m: multiplet; t: triplet

The evidence indicates the structure to be:

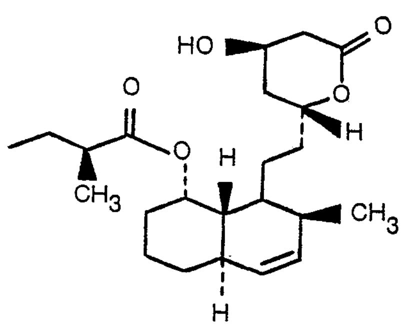

*DESCRIPTION OF THE INVENTION*

We have found that the α-methylbutyryl group in Compound $III_a$ ($R'=CH_3$) and hydro-derivatives, $III_{b-e}$, can be removed cleanly to produce a family of 6(R) - [2 - (8 - hydroxy - 2,6 - dimethylpolyhydronaphthyl - 1) ethyl] - 4(R) - hydroxy - 3,4,5,6 - tetrahydro - 2H - pyran - 2 - ones which are themselves hypocholesterolemic agents and which are extremely useful as intermediates for the preparation of novel esters which are even more potent in this use.

The preparation of the novel alcohols of this invention is carried out by heating the esters $III_{a-e}$ ($R'=CH_3$) with an alkali metal hydroxide such as lithium hydroxide, potassium hydroxide or sodium hydroxide in a protic solvent such as water or alcohols for extended periods. Preferred is lithium hydroxide in water at reflux for about 50—72 hours or under pressure at higher temperatures of 120—180°C for shorter times of 8—24 hours.

The pyranone ring readily opens but the removal of the side chain acyl group is not easily effected. The heating must be prolonged and/or pressure must be used. An inert atmosphere is also helpful. It is quite unexpected that molecules with so many highly sensitive functional centers can withstand the harsh conditions necessary for removal of the highly hindered α-methylbutyryl ester. It is especially unexpected to find the yields high.

In the case of Compounds $III_{a-e}$ ($R'=H$) the saponification of the 8'-esters is much more facile proceeding to completion in about 20 hours to give $IV_{a-e}$ ($R'=H$).

The Compound $IV_a$ (R′=H) is known as ML—236A as reported by Endo et al. in U.S. Patent 3,983,140.

The products are isolated by acidification and extraction with organic solvents which provides the trihydroxy acid form of compounds $IV_{a-e}$. These trihydroxy acids can be relactonized by heating a solution of the acid in an appropriate organic solvent such as toluene or benzene in an apparatus permitting continuous separation of the water formed.

The alcohols which form part of this invention comprise Structures $IV_{a-e}$ (R′=$CH_3$) as well as the trihydroxy acids resulting from opening of the lactone rings.

An alternate synthetic route to the Compounds $IV_{b,c,e}$ comprises the steps of hydrolysis of $III_a$ to $IV_a$ as described herein followed by hydrogenation of $IV_a$ under the conditions described previously for the preparation of $III_{b,c,e}$ to produce $IV_b$, $IV_c$ or $IV_e$ depending on those reaction conditions.

Preparation of Compounds $IV_{a-e}$

The starting materials, the 8′α-hydroxy compounds $IV_{a-e}$ (R′=$CH_3$) are prepared from the various 8′-esters described by Monaghan et al ($III_a$, R′=$CH_3$), Albers-Schonberg et al ($III_d$, R′=$CH_3$) and Patchett et al ($III_{b,c,e}$, R′=$CH_3$) by heating them with lithium hydroxide solution for extended periods. The pyranone ring readily opens but the removal of the side chain acyl group is not easily effected. The heating must be prolonged and/or pressure must be used. An inert atmosphere is also helpful.

In the case of the Compounds $III_{a-e}$ (R′=H) the saponification of the 8′-esters is much more facile proceeding to completion in about 20 hours.

The 8′-hydroxy products are isolated by acidification and extraction with organic solvents which provides the hydroxy acid form, in which the pyranone ring is still opened. These hydroxy acids are relactonized by heating a solution of the acid in an appropriate organic solvent such as benzene or toluene in an apparatus permitting continuous separation of the water formed.

The Compound $IV_a$ (R′=H) is known as ML—236A as reported by Endo et al in U.S. Patent 3,983,140.

In their lactone form, these alcohols are the compounds of Formula $IV_{a-e}$ in Table I and are prepared as described in the following preparations.

Preparation of 6(R) - [2 - (8′(S) - hydroxy - 2′(S),6′(R) - dimethyl - 1′,2′,6′,7′,8′,8′a(R) - hexahydronaphthyl - 1′(S)) - ethyl] - 4(R) - hydroxy - 3,4,5,6 - tetrahydro - 2H - pyran - 2 - one, $IV_a$ (R′=$CH_3$)

A mixture of 8.0 g. (19.78 mmole) of MK—803 ($III_a$, R′=$CH_3$) and 8.31 g (197.8 mmole) of $LiOH.H_2O$ in 600 ml of water was stirred at reflux under a nitrogen atmosphere for 56 hours. The reaction mixture was cooled to 0° and treated, with stirring, with 20 ml of concentrated hydrochloric acid. The mixture was then extracted with three 250-ml portions of ether and the combined extracts were washed succcessively with three 200-ml portions of water and then 200 ml of saturated brine. After drying over $MgSO_4$, this organic solution was filtered and the solvent evaporated *in vacuo* to give an oily residue. This residue was dissolved in 200 ml of toluene and heated at reflux under a nitrogen atmosphere for 2 hours with continuous separation of water to effect relactonization. Evaporation of the toluene and trituration of the residue with hexane gave 5.15 g (81%) of the title compound $IV_a$ (R′=$CH_3$) as a white solid which did not require further purification.

An analytical sample was prepared by recrystallization of a portion of this material from butyl chloride to give white clusters: m.p. 128—131° (vacuum); NMR ($CDCl_3$) δ 0.87 (d, 3, J=7Hz, $CH_3$), 1.16 (d, 3, J=7Hz, $CH_3$), 2.64 (m, 2, pyran $C_3H$'s), 4.27 (brm, 1, naphthalene $C_8H$), 4.37 (m, I, pyran $C_4H$), 4.71 (m, I, pyran $C_6H$), 5.56 (m, I, naphthalene $C_5H$), 5.79 (dd, I, J=6,10 Hz, naphthalene $C_3H$), 6.03 (d, I, J=10 Hz, naphthalene $C_4H$); IR ($CHCl_3$) 3400 (OH), 1725 (C=O), 1240, 1120, 1080 $cm^{-1}$.

*Anal.* Calcd for $C_{19}H_{28}O_4.0.1C_4H_9Cl$ C, 70.67; H, 8.84. Found: C, 70.77; H, 8.75.

Alternative preparation of 6(R) - [2 - [8′(S) - hydroxy - 2′(S),6′(R) - dimethyl - 1′,2′,6′,7′,8′,8′a(R) - hexahydronaphthyl - 1′(S)]ethyl] - 4(R) - hydroxy - 3,4,5,6 - tetrahydro - 2H - pyran - 2 - one, $IV_a$ (R′=$CH_3$)

A suspension of 188 mg (0.463 mmol) of MK—803 ($III_a$, R′=$CH_3$) in 5 ml (5 mmol) of aqueous 1N LiOH solution is shaken for 12 hours at 135° in a 30 ml stainless steel pressure vessel. The cooled reaction mixture is acidified with 1M $H_3PO_4$ and extracted with ethyl acetate. The ethyl acetate solution is dried ($MgSO_4$) and filtered and the solvent is evaporated. The residue is dissolved in 20 ml of toluene which is heated to reflux for 4 hours in a Dean-Stark apparatus to effect relactonization. Evaporation of the toluene gives the title compound.

Preparation of alcohols $IV_a$ (R′=H) and $IV_b$, $IV_c$, $IV_d$, and $IV_e$ (R′=H or $CH_3$)

Following essentially either procedure described above but substituting an equivalent amount of esters $III_a$ (R′=H) or $III_b$, $III_c$, $III_d$, or $III_e$ (R′=H or $CH_3$), for $III_a$ (R′=$CH_3$) used therein the corresponding alcohols $IV_a$ (R′=H), $IV_b$, $IV_c$, $IV_d$ and $IV_e$ (R′=H or $CH_3$) are respectively obtained.

We have found that the 8′-hydroxy compounds of Structure IV can be acylated to give a new class of 8-acyloxy compounds of the structure defined by Formulas I and II and the definitions thereunder. These new compounds are inhibitors of cholesterol synthesis *in vivo.*

The absolute configuration of these compounds is known from X-ray diffraction. Table I provides a convenient tabulation of these structures and their stereochemical relationship. The reference numerals to the various compounds, including those of the various series of polyhydronaphthyl structures, remain the

same throughout these specifications and are so used. Each of the esters $I_{a-e}$ (R'=$CH_3$), of this invention contains seven or eight chiral centers. The relative and absolute configuration of these asymmetric centers is as depicted in Table I. More specifically, for ester $I_a$ (R'=$CH_3$), the Cahn, Ingold, Prelog designations for the absolute configurations are 4(R), 6(R), 1'(S), 2'(S), 6'(R), 8'(S) and 8a'(R) [R. S. Cahn, C. Ingold and V. Prelog, *Angew. Chem. Int. Ed., 5,* 385 (1966)].

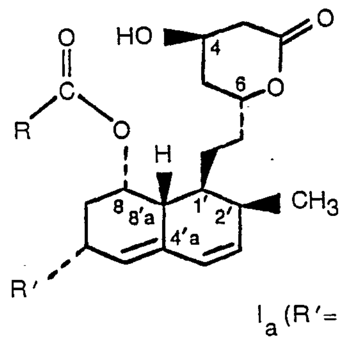

$I_a$ (R'=$CH_3$)

As is indicated in the formulas $I_{a-e}$, all of these compounds have the same spatial orientation of groups at each chiral carbon atom and therefore belong to the same stereochemical series. The R—S designation for each center may not be identical to that found for the ester $I_a$ (R'=$CH_3$) because of the details of the sequence rules used for determining that designation. In the two esters $I_d$ and $I_e$ which have an additional chiral carbon atom not present in ester $I_a$, the hydrogen atom at 4a' is in the down (or α) orientation as depicted in Table I, giving a *trans* ring junction.

TABLE I

THE COMPOUNDS OF THIS INVENTION AND THEIR STEREO-RELATIONSHIP

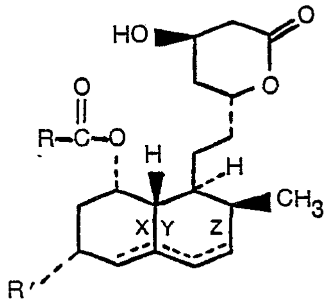

$I_{a-e}$

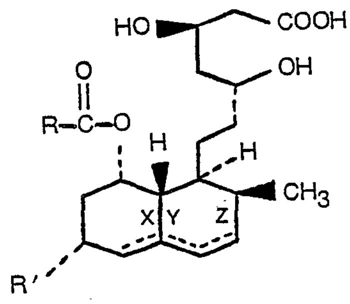

$II_{a-e}$

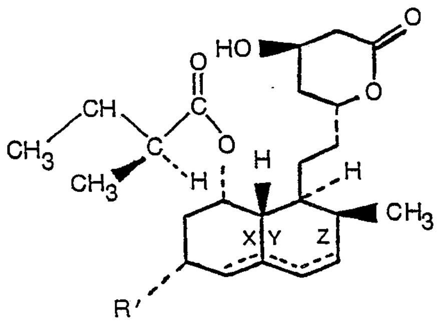

$III_{a-e}$

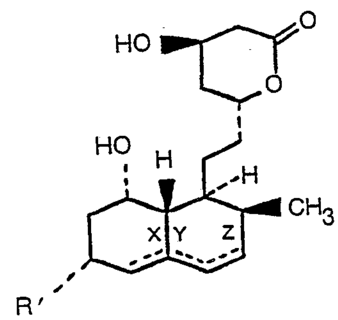

$IV_{a-e}$

R' = H or $CH_3$

STEREOCHEMISTRY OF THE HYDRONAPHTHYL SERIES

| Series | Double Bonds Present | Structure |
|---|---|---|
| a | X and Z | 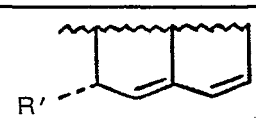 |
| b | X | 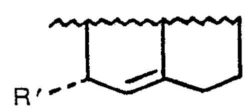 |
| c | Y | 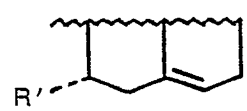 |
| d | Z | 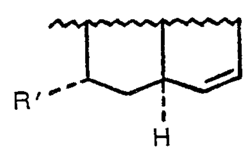 |
| e | None | 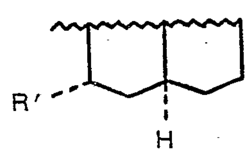 |

The 8'-acyloxy compounds of this invention are useful as antihypercholesterolemic agents for the treatment of atherosclerosis, hyperlipemia and like diseases in humans. They may be administered orally or parenterally in the form of a capsule, a tablet, an injectable preparation or the like. It is usually desirable to use the oral route. Doses may be varied, depending on the age, severity, body weight and other conditions of human patients, but daily dosage for adults is within a range of from about 2 mg to 2000 mg (preferably 10 to 100 mg) given in three or four divided doses. Higher doses may be favorably applied as required.

The compounds of this invention also have useful anti-fungal activities. For example, they may be used to control strains of *Penicillium sp., Aspergillus niger, Cladosporium sp., Cochliobolus miyabeanus* and *Helminthosporium cynodnotis.* For those utilities they are admixed with suitable formulating agents, powders, emulsifying agents or solvents such as aqueous ethanol and sprayed or dusted on the plants to be protected.

The preparation of the compounds of this invention is described in Flow Sheet A.

FLOW SHEET A

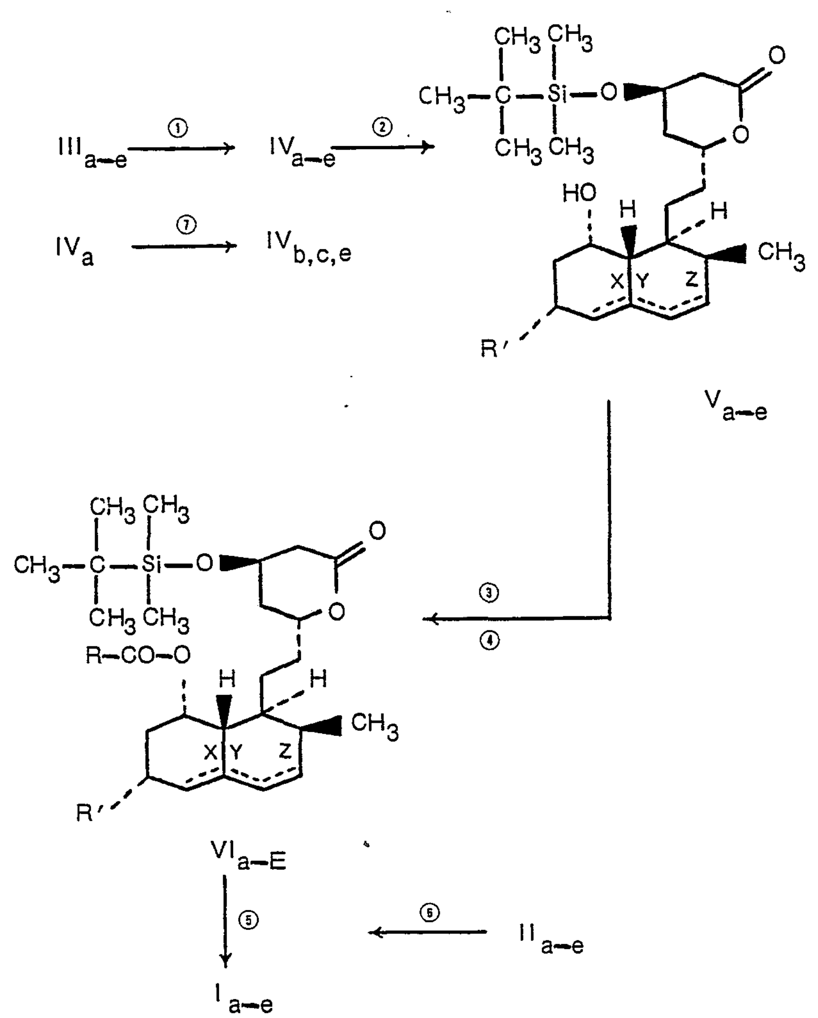


*Definitions*

X, Y, Z, R and R' as defined in specification and series a-e as defined in Table I.

*Reactions*

1) Lithium hydroxide, heat, acidify and lactonize.

2) t-Butyldimethylchlorsilane and imidazole in DMF at ambient temperatures in an inert atmosphere.

3) Treatment with RCOCl and 4-dimethylaminopyridine in pyridine solution preferably under inert atmosphere.

4) Treatment with RCOOH and N,N'-dicyclohexylcarbodiimide and 4-pyrrolidinopyridine in dichloromethane, preferably under an inert atmosphere.

5) Three equivalents of tetrabutylammonium fluoride and four equivalents of acetic acid per equivalent of ester in THF, preferably in an inert atmosphere.

6) Aqueous alkali followed by careful acidification with dilute acid.

7) See Reactions and Reagents and Flow Sheet for synthesis of $III_{b,c,e}$.

In the novel process of this invention the 4-hydroxyl on the pyranone ring, of alcohols $IV_{a-e}$ is first protected with a t-butyldimethylsilyl group by reaction with t-butyldimethylchlorosilane in an inert atmosphere at ambient temperatures in the presence of an acid acceptor such as imidazole to provide the protected alcohols $V_{a-e}$. The 8-hydroxyl on the polyhydronaphthyl ring is then acylated in one of two ways. The first comprises treatment with the acid chloride of the desired acyl group in pyridine in the presence of 4-dimethylaminopyridine as a catalyst. The second comprises treatment of the 8'-polyhdyronaphthol with the free acid of the desired acyl group and a carbodiimide such as N,N'-dicyclohexylcarbodiimide with 4-pyrrolidinopyridine as a catalyst in dichloromethane. These procedures give the protected esters $VI_{a-e}$. The removal of the silyl protecting group from the 4-hydroxyl of the pyranone ring is then carried out, using three equivalents of tetrabutylammonium fluoride and four equivalents of acetic acid per equivalent of esters $VI_{a-e}$, to give the desired compounds $I_{a-e}$. The ratio of reagents in this last reaction is critical to the yield of the process and the purity of the products.

The acyl groups thus put on the 8'-hydroxyl are those in which R in $I_{a-e}$ is:

1) $C_{1-)0}$ straight, or branched chain alkyl except (S)-2-butyl,

2) $C_{3-10}$ cycloalkyl,

3) $C_{2-10}$ alkenyl,

4) $C_{1-10}$ $CF_3$-substituted alkyl,

5) phenyl,

6) halophenyl, wherein halo is chloro, fluoro, bromo or iodo,

7) phenyl-$C_{1-3}$ alkyl,

8) substituted phenyl-$C_{1-3}$ alkyl in which the substituent is halo, such as fluoro, chloro, bromo, or iodo, $C_{1-3}$ alkyl or $C_{1-3}$ alkoxy.

It is preferred that R' be $CH_3$.

Preferred definitions of R, are:

$C_{2-5}$ straight chain alkyl,

$C_{3-10}$ branched chain alkyl except (S)-2-butyl,

$C_{3-10}$ cycloalkyl,

$C_{3-10}$ alkenyl in which the unsaturation is not in conjugation with the carbonyl, especially

$C_{3-10}$ branched chain alkyl except (S)-2-butyl.

Preferred species are those wherein R is 1,1-diethylpropyl or 1-ethyl-1-methylpropyl. And it is especially preferred that none of X, Y, or Z is a double bond.

Compounds $I_{a-e}$ can be hydrolyzed with bases such as NaOH to yield the salts such as the sodium salt of Compounds $II_{a-e}$. The use of bases with other pharmaceutically acceptable cations affords salts of those cations. Careful acidification of the salts affords the hydroxy acids $II_{a-e}$ which revert to Compounds $I_{a-e}$ at acidic pH. Treating Compound $I_{a-e}$ under acidic or basic catalysis with methanol, ethanol, propanol, or butanol or with phenyl-, dimethylamino-, or acetylamino-alkanols yields the corresponding esters of Compounds $II_{a-e}$ which also form a part of this invention.

The pharmaceutically acceptable salts of this invention include those formed from cations such as sodium, potassium, aluminum, calcium, lithium, magnesium, zinc and tetramethylammonium as well as those salts formed from amines such as ammonia, ethylenediamine, N-methylglucamine, lysine, arginine, ornithine, choline, N,N'-dibenzylethylenediamine, chloroprocaine, diethanolamine, procaine, N-benzylphenethylamine, 1-p-chlorobenzyl-2-pyrrolidine-1'-yl-methylbenzimidazole, diethylamine, piperazine, and tris(hydroxymethyl)aminomethane.

Example 1

6(R) - [2 - (8'(S) - 2",2" - dimethylpropanoyloxy - 2'(S),6'(R) - dimethyl - 1',2',6',7',8',8'a(R) - hexahydronaphthyl - 1'(S)) - ethyl] - 4(R) - hydroxy - 3,4,5,6 - tetrahydro - 2H - pyran - 2 - one

*Step A:* Preparation of 6(R) - [2 - (8'(S) - hydroxy - 2'(S) - 6'(R) - dimethyl - 1',2',6',7',8',8'a(R) - hexahydronaphthyl - 1'(S))ethyl] - 4(R) - (dimethyl - tert - butylsilyloxy) - 3,4,5,6 - tetrahydro - 2H - pyran - 2 - one, $V_a$ ($R'=CH_3$)

A mixture of the alcohol $IV_a$ ($R'=CH_3$) (18.3 g, 57.1 mmol), 21.5 g (142.8 mmol) of tert-butyldimethylchlorosilane and 19.4 g (285.6 mmol) of imidazole in 200 ml of N,N-dimethylformamide was stirred at 20° under a nitrogen atmosphere for 18 hours. The reaction mixture was then diluted with 1500 ml of ether and washed successively with water, 2% aqueous hydrochloric acid, water and saturated sodium bicarbonate. The ether solution was dried over $MgSO_4$, filtered and reduced to a volume of 1 L. After addition of 600 ml of hexane, the volume was reduced to 600 ml on a steam bath. The product crystallized at room temperature; after isolation and air drying this provided 13.7 g of a white cottony solid. The mother liquors were reduced to 250 ml and a second crop of crystals was isolated after this solution stood at 0° overnight. The combined yield was 17.13 g (69%) of the title compound as a white cottony solid: mp 142—144° (vac); NMR ($CDCl_3$) δ 0.10 (s, 6, $(CH_3)_2Si$), 0.90 (s, 9, $(CH_3)_3CSi$), 1.19 (d, 3, J=7Hz, $CH_3$), 2.58 (d, 2, J=4Hz, pyran $C_3H$'s), 4.3 (m, 2, pyran $C_4H$ and naphthalene $C_8H$) 4.70 (m, l, pyran $C_6H$), 5.57 (m, l, naphthalene $C_5H$), 5.58

(dd, 1, J=6,10Hz, naphthalene $C_3H$), 6.03 (d, I, J=10Hz, naphthalene $C_4H$).
*Anal.* Calcd. for $C_{25}H_{42}O_4Si$: C, 69.08, H, 9.74. Found: C, 69.46; H, 9.83.

*Step B:* Preparation of 6(R) - [2 - (8′(S) - 2″,2″ - dimethylpropanoyloxy - 2′(S),6′(R) - dimethyl - 1′,2′,6′,7′,8′,8′a(R) - hexahydronaphthyl - 1′(S))ethyl] - 4(R) - (dimethyl - tert - butylsilyloxy) - 3,4,5,6 - tetrahydro - 2H - pyran - 2 - one, $VI_a$ (R′=$CH_3$)

A solution of 6.0 g (13.8 mmol) of the alcohol $V_a$ (R′=$CH_3$) from Step A and 200 mg of 4-dimethylaminopyridine in 50 ml of pyridine was cooled to 0° under a nitrogen atmosphere. To this stirred solution was added 6.8 ml (6.65 g, 55.2 mmol) of pivaloyl chloride over 15 minutes. The reaction mixture was stirred at 0° for 1 hour and then at 20° for 4 days. The reaction mixture was diluted with 750 ml of ether and washed with 2% aqueous hydrochloric acid until the wash was acidic and then with saturated $NaHCO_3$ solution. After drying over $MgSO_4$ the solution was filtered and evaporated to give 7.81 g of the title compound as a light orange oil: NMR ($CDCl_3$) δ 0.09 (s, 6($CH_3$)$_2$Si), 0.88 (s, 9, $(CH_3)_3CSi$), 1.28 (s, 9, $(CH_3)_3CCO_2$—), 2.57 (d, 2, J=4Hz, pyran $C_3H$'s), 4.32 (m, I, pyran $C_4H$), 4.63 (m, I, pyran $C_6H$), 5.34 (m, I, naphthalene $C_8H$), 5.54 (m, I, naphthalene ($C_5H$), 5.78 (dd, I, J=6, 10Hz, naphthalene $C_3H$), 6.03 (d, I, J=10Hz, naphthalene $C_4H$).

Employing the procedure substantially as described in Example 1, Step B, but substituting for the pivaloyl chloride used therein, an equimolecular amount of the acid chloride of structure R—COCl described in Table II, there are prepared the esters of structure $VI_a$ (R′=$CH_3$) also described in Table II.

TABLE II

| $R-\overset{O}{\overset{\|}{C}}-O$ | NMR($CDCl_3$, δ) |
|---|---|
| 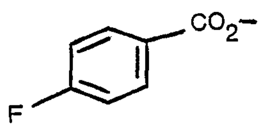 | 7.10(t,2,J=8Hz,p—FPh—)<br>8.03(dd,2,J=5,8Hz,p—FPh—) |
| $CH_3CO_2-$ | 2.02(s,3,$CH_3CO_2$—) |
| 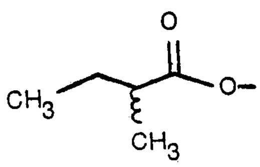 | 1.19(d,J=7Hz,α—$CH_3$ ester<br>1.21(d,J=7Hz,α—$CH_3$ ester)<br>Total 3H |
| $(CH_3)_2CHCH_2CO_2-$ | 0.83(d,6,J=6Hz,$(CH_3)_2CH$—) |
| $(CH_3)_2CHCO_2-$ | 1.13(d,6,J=6Hz$(CH_3)_2CH$) |
| $CH_3(CH_2)_3CO_2-$ | 0.95(t,3,J=7Hz,$CH_3-(CH_2)_3-$ |
| 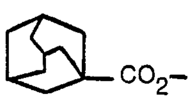 | 1.60–2.08 (m,15,Adamantyl) |
| $CH_3(CH_2)_6CO_2-$ | |
| $C_6H_{11}CO_2-$ | |
| $CH_2{=}CH-CO_2-$ | |
| $CF_3(CH_2)_2CO_2-$ | |

TABLE II (Continued)

| 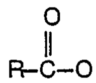 | NMR($CDCl_3$, δ) |
|---|---|
| $C_6H_5CO_2$– | |
| 4–$ClC_6H_4CO_2$– | |
| 2,4–$F_2C_6H_3CO_2$– | |
| $C_6H_5(CH_2)_3CO_2$– | |
| 4–$FC_6H_4CH_2CO_2$– | |
| 2,4–$F_2C_6H_3CH_2CO_2$– | |
| 4–$ClC_6H_4CH_2CO_2$– | |
| 4–$FC_6H_4(CH_2)_3CO_2$– | |
| 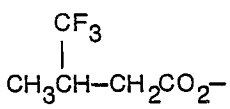 | |
| 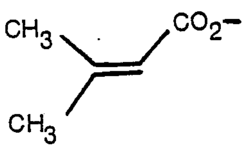 | |
| 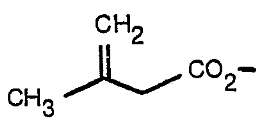 | |
| $CH_3(CH_2)_8CO_2$– | |
| 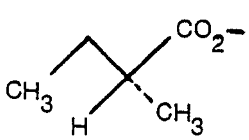 | |

*Step C:*

Preparation of 6(R)-[2-(8′(S)-2″,2″-dimethylpropanoyloxy-2′(S),6′(R)-dimethyl-1′,2′,6′,7′,8′,8′a(R)-hexahydronaphthyl-1′(S))ethyl]-4(R)-hydroxy-3,4,5,6-tetrahydro-2H-pyran-2-one, $I_a$ (R′=$CH_3$)

To a solution of 10.0 g (31.7 mmol) of $Bu_4N^+F^-.3H_2O$ and 2.4 ml (2.5 g, 42.3 mmol) of acetic acid in 50 ml of tetrahydrofuran was added 7.81 g (13.8 mmol) of the silyl ether $VI_a$ (R′=$CH_3$) from Step B in 50 ml tetrahydrofuran. This mixture was stirred at 20° under a nitrogen atmosphere for 18 hours. The reaction mixture was diluted with 700 ml of ether and washed successively with 2% aqueous hydrochloric acid, water and saturated aqueous $NaHCO_3$. The organic solution was dried ($MgSO_4$) and filtered. Evaporation of the solvent left 6.45 g of an off-white solid. This material was crystallized from 100 ml of butyl chloride and the isolated crystals were dried at 35°/0.01 mm for four hours to give 4.0 g (72%) of the title compound as nearly white needles: mp 167.5—170.5° (vac); NMR ($CDCl_3$) δ 0.88 (d, 3, J=7Hz, $CH_3$), 1.08 (d, 3, J=7Hz, $CH_3$), 1.19 (s, 9, $(CH_3)_3C$), 2.67 (d, 2, J=4Hz, pyran $C_3H$'s), 4.39 (m, 1, pyran $C_4H$), 4.65 (m, 1, pyran $C_6H$), 5.36 (m, 1, naphthalene $C_8H$) 5.55 (m, 1, naphthalene $C_5H$), 5.80 (dd, 1, J=6, 10Hz, naphthalene $C_3H$), 6.04 (d, 1,

J=10Hz, naphthalene $C_4H$); HPLC (4.6 mm. × 25 cm Partisil 10 PAC, 10% isopropanol/hexane, 4 ml/min) retention time 4.4 min.

*Anal.* Calcd. for $C_{24}H_{36}O_5$: C, 71.25; H, 8.97. Found: C, 71.40; H, 8.93.

Employing the procedure of Example 1, Step C, but substituting for the 2,2-dimethylpropanoyloxy-silyl ether Compound $VI_a$ ($R'=CH_3$) used therein, an equimolecular amount of the other esters of structure $VI_a$ ($R'=CH_3$) described in Table II, there are prepared the esters of structure $I_a$ ($R'=CH_3$), described in Table III.

TABLE III

| $RCO_2-$ | Formula | MP(°C) |
|---|---|---|
| 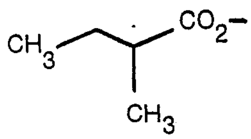 | $C_{24}H_{36}O_5$ | 139–148 |
| 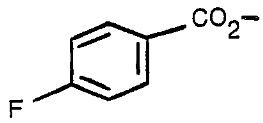 | $C_{26}H_{31}FO_5$ | 119.5–120.5 (vac) |
| $(CH_3)_2CHCH_2CO_2-$ | $C_{24}H_{36}O_5$ | 126–128 |
| $(CH_3)_2CHCO_2-$ | $C_{23}H_{34}O_5$ | 144–147 |
| $CH_3(CH_2)_3CO_2-$ | $C_{24}H_{36}O_5$ | |
| $CH_3CO_2-$ | $C_{21}H_{30}O_5.0.1C_4H_9$ | 153–156 (vac) |
| 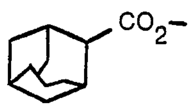 | $C_{30}H_{42}O_5.0.05C_6H_{12}$ | 155–158 |
| $CH_3(CH_2)_6CO_2-$ | | |
| $C_6H_{11}CO_2-$ | | |
| $CH_2=CH-CO_2-$ | | |
| $CF_3(CH_2)_2CO_2-$ | | |
| $C_6H_5CO_2-$ | | |
| $4-ClC_6H_4CO_2-$ | | |
| $2,4-F_2C_6H_3CO_2-$ | | |
| $C_6H_5(CH_2)_3CO_2-$ | | |
| $4-FC_6H_4CH_2CO_2-$ | | |
| $2,4-F_2C_6H_3CH_2CO_2-$ | | |
| $4-ClC_6H_4CH_2CO_2-$ | | |
| $4-FC_6H_4(CH_2)_3CO_2-$ | | |

TABLE III (Continued)

| $RCO_2-$ | Formula | MP(°C) |
|---|---|---|
| 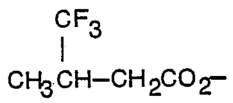 | | |
| 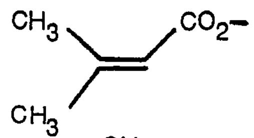 | | |
| 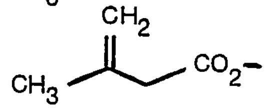 | | |
| $CH_3(CH_2)_8CO_2-$ | | |
| 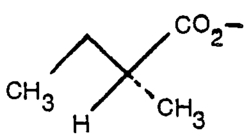 | | |

Example 2

6(R)-[2-(8′(S)-phenylacetoxy-2′(S),6′(R)-dimethyl-1′,2′,6′,7′,8′,8a′(R)-hexahydronaphthyl-1′(S))ethyl]-4-(R)-hydroxy-3,4,5,6-tetrahydro-2H-pyran-2-one

*Step A:*

Preparation of 6(R)-[2-(8′(S)-phenylacetoxy-2′(S),6′(R)-dimethyl-1′,2′,6′,7′,8′,8′a(R)-hexahydronaphthyl-1′(S))ethyl]-4(R)-dimethyl-tert-butylsilyloxy)-3,4,5,6-tetrahydro-2H-pyran-2-one, $VI_a$ ($R'=CH_3$)

A solution of 434 mg (1.0 mmol) of the alcohol $V_a$ ($R'=CH_3$) from Example 1, Step A, 204 mg (1.5 mmol) of phenylacetic acid, and 309 mg (1.5 mmol) of N,N′-dicyclohexylcarbodiimide in 10 ml of dichloromethane was treated with 22 mg (0.15 mmol) of 4-pyrrolidinopyridine and stirred at 20° under a nitrogen atmosphere. After 3 days the solvent was removed *in vacuo* and the residue was suspended in 25 ml of ether and filtered. Evaporation of the filtrate gave a viscous oil which was chromatographed on a 3 × 15 cm. column of silica gel (230—400 mesh). Elution (under air pressure) with ether-hexane (1:1, v:v) gave 460 mg (83%) of the title compound as a viscous oil: NMR ($CDCl_3$) δ 0.10 (s, 6, $(CH_3)_2Si$), 0.90 (s, 9, $(CH_3)_3CSi$), 3.58 (s, 2, $PhCH_2$—) 5.34 (m, 1, naphthalene $C_8H$), 7.30 (s, 5, Ph).

Employing the procedure of Example 2, Step A, but substituting for the phenylacetic acid used therein, an equimolecular amount of the organic acids of structure R—COOH described in Table IV there are produced the esters of structure $VI_a$ ($R'=CH_3$) also described in Table IV.

TABLE IV

| 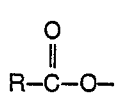 | NMR($CDCl_3$, δ) |
|---|---|
| 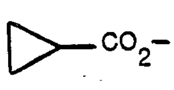 | 0.78–1.02(m,4,cyclopropane) |
| 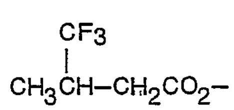 | 1.04(d,3,J=7Hz,$C\underline{H}_3CHCF_3$) |

TABLE IV (Continued)

| 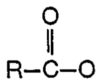 | NMR($CDCl_3$, $\delta$) |
|---|---|
| 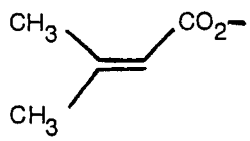 | 1.88(s,3,$CH_3C{=}C$)<br>2.17(d,3,J=2Hz,$CH_3C{=}C$)<br>5.68 (brs,1,C=CH−) |
| 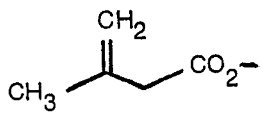 | 1.80 (s,3,$CH_3C{=}C$)<br>4.86,4.92(s,2,$CH_2{=}C$) |
| $CH_3(CH_2)_8CO_2^-$ | 0.87(m,3,$C\underline{H}_3(CH_2)_8CO_2-$)<br>1.25(m,14,$CH_3(C\underline{H}_2)_7CH_2CO_2-$) |
| 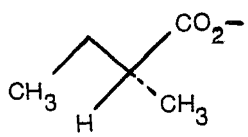 | |
| 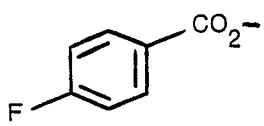 | |
| $CH_3CO_2-$ | |
| 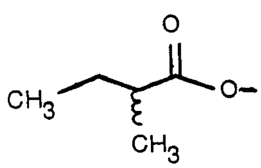 | |
| $(CH_3)_2CHCH_2CO_2-$ | |
| $(CH_3)_2CHCO_2-$ | |
| $CH_3(CH_2)_3CO_2-$ | |
| 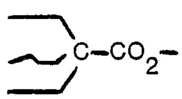 | |
| 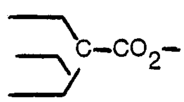 | |
| 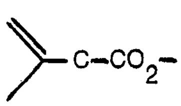 | |
| 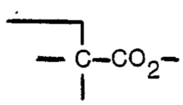 | |

TABLE IV (Continued)

| $R-\overset{\overset{O}{\|}}{C}-O$ | NMR($CDCl_3$, $\delta$) |
|---|---|
| $CH_3(CH_2)_6CO_2-$ | |
| $C_6H_{11}CO_2-$ | |
| $CH_2=CH-CO_2-$ | |
| $CF_3(CH_2)_2CO_2-$ | |
| $C_6H_5CO_2-$ | |
| $4-ClC_6H_4CO_2-$ | |
| $2,4-F_2C_6H_3CO_2-$ | |
| $C_6H_5(CH_2)_3CO_2-$ | |
| $4-FC_6H_4CH_2CO_2-$ | |
| $2,4-F_2C_6H_3CH_2CO_2-$ | |
| $4-ClC_6H_4CH_2CO_2-$ | |
| $4-FC_6H_4(CH_2)_3CO_2-$ | |

*Step B:*
Preparation of 6(R)-[2-(8′(S)-phenylacetoxy-2′(S),6′(R)-dimethyl-1′,2′,6′,7′,8′,8′a(R)-hexahydronaphthyl-1′(S))ethyl]-4(R)-hydroxy-3,4,5,6-tetrahydro-2H-pyran-2-one, $I_a$ ($R'=CH_3$)

Employing the procedure substantially as described in Example 1, Step C, but substituting for the propanoyloxy compound used therein an equimolar amount of the phenylacetoxy compound from Example 2, Step A, there is produced the title compound, m.p. 109—112°C.

Employing the other esters, $VI_a$ ($R'=CH_3$) described in Example 2, Step A, (Table IV) and following the procedure of Example 2, Step B, there are produced the esters of structure $I_a$ ($R'=CH_3$) described in Table V.

TABLE V

| $RCO_2$ | Formula | m.p. (°C) |
|---|---|---|
| 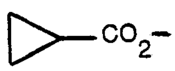 | $C_{23}H_{32}O_5$ | 116–119 |
| $CH_3\overset{\overset{CF_3}{\|}}{C}HCH_2CO_2-$ | $C_{24}H_{33}F_3O_5$ | 110–113 |
| 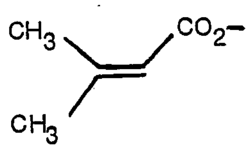 | $C_{24}H_{34}O_5$ | 113–118 |
| 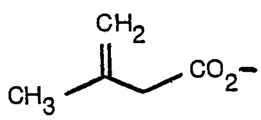 | $C_{24}H_{34}O_5$ | 116–119 |
| $CH_3(CH_2)_8CO_2-$ | $C_{29}H_{46}O_5$ | (wax) |

TABLE V (Continued)

| $RCO_2$ | Formula | m.p. (°C) |
|---|---|---|
| 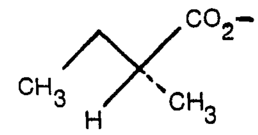 | $C_{24}H_{36}O_5$ | 126–129 |
| 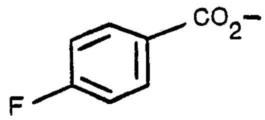 | | |
| $CH_3CO_2-$ | | |
| 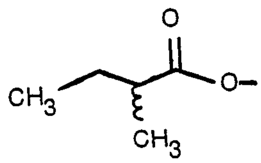 | | |
| $(CH_3)_2CHCH_2CO_2-$ | | |
| $(CH_3)_2CHCO_2-$ | | |
| $CH_3(CH_2)_3CO_2-$ | | |
| 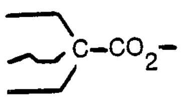 | | |
| 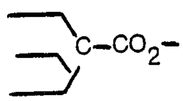 | | |
| 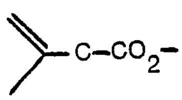 | | |
| 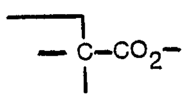 | | |
| $CH_3(CH_2)_6CO_2-$ | | |
| $C_6H_{11}CO_2-$ | | |
| $CH_2{=}CH-CO_2-$ | | |
| $CF_3(CH_2)_2CO_2-$ | | |
| $C_6H_5CO_2-$ | | |
| $4-ClC_6H_4CO_2-$ | | |
| $2,4-F_2C_6H_3CO_2-$ | | |
| $C_6H_5(CH_2)_3CO_2-$ | | |
| $4-FC_6H_4CH_2CO_2-$ | | |
| $2,4-F_2C_6H_3CH_2CO_2-$ | | |
| $4-ClC_6H_4CH_2CO_2-$ | | |
| $4-FC_6H_4(CH_2)_3CO_2-$ | | |

Example 3

6(R)-[2-(8'(S)-2''-ethyl-2''-methylbutyryloxy-2'(S)-6'(R)-dimethyl-1',2',6',7',8',8'a(R)-hexahydronaphthyl-1'(S))-ethyl]-4(R)-hydroxy-3,4,5,6-tetrahydro-2H-pyran-2-one

*Step A:*

Preparation of 6(R)-[2-(8'(S)-2''-ethyl-2''-methylbutyryloxy-2'(S)-6'(R)-dimethyl-1',2',6',7',8',8'a(R)-hexahydronaphthyl-1'(S))ethyl]-4(R)-(dimethyl-tert-butylsilyloxy)-3,4,5,6-tetrahydro-2H-pyran-2-one $VI_a$ ($R'=CH_3$)

3.0 g of 2-ethyl-2-methylbutyryl chloride (20 mmol) was added to a magnetically stirred solution of 2.17 g (5 mmol) of alcohol $V_a$ ($R'=CH_3$) and 74 mg of 4-pyrrolidino pyridine in 20 ml of pyridine. This reaction mixture was stirred at 100°C under an atmosphere of $N_2$ for nine hours. The reaction mixture was diluted with 500 ml ether and washed with 1N HCl until the wash was acidic and then with brine (3 × 50 ml). After drying over $MgSO_4$, the solution was filtered and evaporated to give 4.2 g of a brown oil. This oil was chromatographed on a 6 × 15 cm column of silica gel (230—400 mesh). Elution (under air pressure) with ether-hexane (1:1, v:v) gave 2.6 g (95%) of the title compound as a viscous yellow oil: NMR ($CDCl_3$) δ 0.08 (s, 6, $(CH_3)_2Si$), 0.9 (s, 9, $(CH_3)_3$ CSi), 2.57 (d, 2, J=4Hz, pyran $C_3H$'s), 4.30 (m, 1, pyran $C_4H$), 4.63 (m, 1, pyran $C_6H$), 5.42 (m, 1, naphthalene $C_8H$), 5.53 (m, 1, naphthalene $C_5H$), 5.78 (dd, 1, J=6, Hz, 10Hz, naphthalene $C_3H$), 6.03 (d, 1, J=10Hz, naphthalene $C_4H$).

Employing the procedure substantially as described in Example 3, Step A, but substituting for the 2-ethyl-2-methylbutyryl chloride used therein, an equimolecular amount of the acid chlorides of structure R—COCl, described in Table VI, there are produced the esters of structure $VI_a$ ($R'=CH_3$) also described in Table VI.

TABLE VI

| R—CO | NMR($CDCl_3$,) |
|---|---|
| 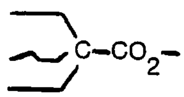 | 0.87(m,9,$C\underline{H}_3CH_2CH_2(C\underline{H}_3CH_2)_2CCO_2$) |
| 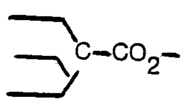 | 0.78(t,9,J=7Hz, $(C\underline{H}_3CH_2)_3CCO_2$)<br>1.48(q,6,J=7Hz, $(CH_3C\underline{H}_2)_3CCO_2$) |
| 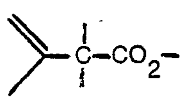 | 1.28(s,6,$(CH_3)_2CCO_2$)<br>2.20(s,3,$C\underline{H}_3-C=CH_2$)<br>3.86(m,2,$CH_2=C$) |
| 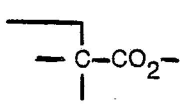 | 1.12(s,6,$(CH_3)_2CCO_2$)<br>0.83(t,3,($C\underline{H}_3CH_2CCO_2$) |

*Step B:*

Preparation of 6(R)-[2-(8'(S)-2''-ethyl-2''-methylbutyryloxy-2'(S)-6'(R)-dimethyl-1',2',6',7',8',8'a-(R)-hexahydronaphthyl-1'(S))ethyl]-4(R)-hydroxy-3,4,5,6-tetrahydro-2H-pyran-2-one

Employing the procedure substantially as described in Example 1, Step C, or Example 2, Step B, but

employing as starting material the silyl ether compound from Example 3, Step A, there is produced the title compound, m.p. 111—113°C ($C_{26}H_{40}O_5$).

Similarly prepared are the esters of structure $I_a$ described in Table VII, employing as starting materials the other esters $VI_a$ (R'=$CH_3$) described in Table VI.

TABLE VII

| $RCO_2-$ | Formula | m.p. (°C) |
|---|---|---|
| 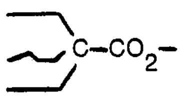 | $C_{28}H_{44}O_5$ | 81–83 |
| 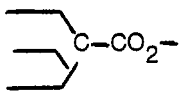 | $C_{27}H_{42}O_5$ | 129–132 |
| 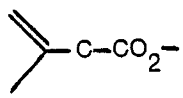 | $C_{26}H_{38}O_5$ | 75–78 |
| 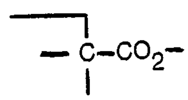 | $C_{25}H_{38}O_5$ | 135–138 |

Employing the procedures of Example 1, Step A, followed by Example 1, Steps B and C, or Example 2 or 3, Steps A and B, but substituting for the diol of structure $IV_a$ (R'=$CH_3$) in Example 1, Step A, the corresponding diols of structure $IV_a$ (R'=H) or $IV_{b,c,d,}$ or $_e$ (R'=H, or $CH_3$), there are produced in sequence the silyl ethers of structures $V_a$ (R'=H) or $V_{b,c,d,}$ and $_e$ (R'=H, or $CH_3$), the esters of structure $VI_a$ (R'=H) or $VI_{b,c,d,}$ and $_e$ (R'=H, or $CH_3$), and the novel esters of structures $I_a$ (R'=H) or $I_{b,c,d}$ and $_e$ (R'=H or $CH_3$) in accordance with Flow Sheet A, wherein

$$R—\overset{O}{\overset{\|}{C}}O$$

of the 8'-alkanoyl group is:

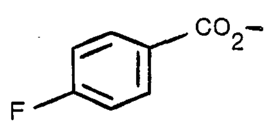

$4-ClC_6H_4CO_2-$

$CH_3CO_2-$

$2,4-F_2C_6H_3CO_2-$

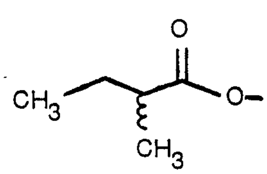

$C_6H_5(CH_2)_3CO_2-$

$(CH_3)_2CHCH_2CO_2-$

$4-FC_6H_4CH_2CO_2-$

$(CH_3)_2CHCO_2-$

$2,4-F_2C_6H_3CH_2CO_2-$

$CH_3(CH_2)_3CO_2-$

$4-ClC_6H_4CH_2CO_2-$

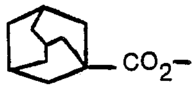

4-$FC_6H_4(CH_2)_3CO_2$−

$CH_3(CH_2)_6CO_2$−

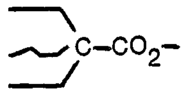

$C_6H_{11}CO_2$−

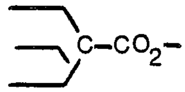

$CH_2$=CH−$CO_2$−

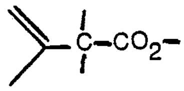

$CF_3(CH_2)_2CO_2$−

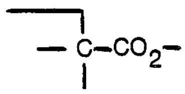

$C_6H_5CO_2$−

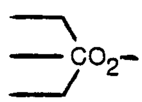

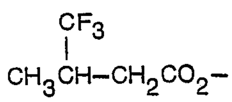

$CH_3(CH_2)_8CO_2$−

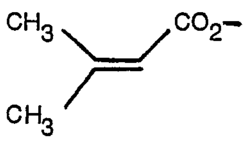

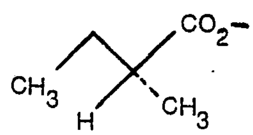

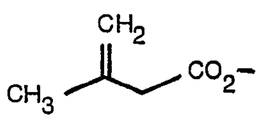

Example 4

Preparation of 6(R)-{2-[8(S)(2″-ethyl-2″-methylbutyryloxy)-2′(S),6′(S)-dimethyl-1′,2′,3′,4′,4′a(S),5′,6′,7′,8′,8′a(S)-decahydronaphthyl-1′(S)]ethyl}-4(R)hydroxy-3,4,5,6-tetrahydro-2H-pyran-2-one, $I_e$ (R′=$CH_3$)

*Step A:*

Preparation of 6(R)-[2-(8′(S)hydroxy-2′(S),6′(S)dimethyl-1′,2′,3′,4′,4′a(S),5′,6′,7′,8′,8′a(S)-decahydronaphthyl-1′(S))ethyl]-4(R)-hydroxy-3,4,5,6-tetrahydro-2H-pyran-2-one $IV_e$ (R′=$CH_3$)

A solution of 2.0 g (6.2 mmol) of the alcohol $IV_a$ (R′=$CH_3$) in 100 ml of ethyl acetate was hydrogenated in the presence of platinum oxide (1 g) at 40 lbs. pressure until an uptake of two mole equivalents of hydrogen was observed. The catalyst was removed by filtration and the filtrate was evaporated to dryness to provide a white solid (1.9 g) which was chromatographed on a 6 × 20 cm column of silica gel (230—400 mesh). Elution (under air pressure) with acetone-methylene chloride (3:7, v:v) gave 1.0 g (50%) of the title compound as a colorless solid.

An analytical sample was prepared by recrystallization of a portion of the material from chloroform to give a white cottony solid: m.p. 166—8°.

*Step B:*

Preparation of 6(R)-[2-(8'(S)-hydroxy-2'(S),6'(S)-dimethyl-1',2',3',4',4'a(S),5',6',7',8',8'a(S)-decahydronaphthyl-1'(S)-ethyl]-4(R)-(dimethyl-tert-butylsilyloxy)3,4,5,6-tetrahydro-2H-pyran-2-one, $V_e$ (R'=$CH_3$)

A solution of the alcohol $IV_3$ (R'=$CH_3$) (1.0 g, 3.1 mmol), imidazole (1.05 g, 15.4 mmol) and tert-butyldimethylchlorosilane (1.16 g, 7.7 mmol) in 20 ml of N,N-dimethyl formamide was stirred at 20°C under a nitrogen atmosphere for 18 hours. The reaction solution was diluted with 200 ml of ether and washed successively with water, 2% aqueous hydrochloric acid and brine. The ether solution was dried over $MgSO_4$ and evaporated to provide a white solid (1.8 g) which was chromatographed on a 6 × 20 cm column of silica (230—400 mesh). Elution under air pressure with acetone:methylene chloride (1:19, v:v) gave 1.0 g (74%) of the title compound as a white solid: m.p. 136—138°C.

*Step C:*

Preparation of 6(R)-{2-[8'(S)(2''-ethyl-2''-methylbutyryloxy)-2'(S),6'(S)-dimethyl-1',2',3',4',4'a(S),5',6',7',8',8'a(S)-decahydronaphthyl-1'(S)-ethyl}-4(R)(dimethyl-tert-butylsilyloxy)-3,4,5,6-tetrahydro-2H-pyran-2-one $VI_e$ (R'=$CH_3$)

By substituting an equimolar amount of alcohol $V_e$ (R'=$CH_3$) for alcohol $V_a$ (R'=$CH_3$) in Step A of Example 3 and following the procedure for Step A there was obtained a corresponding amount of the title compound, $VI_e$ (R'=$CH_3$) as a yellow oil. NMR ($CDCl_3$) 0.08 (S, 6, $(CH_3)_2Si$), 0.90 (S, 9, $(CH_3)_3CSi$), 1.13 (S, 6, $(CH_3)_2CO_2$), 2.63 (m, 2, pyran $C_3H$'s), 4.33 (m, 1, pyran $C_4H$), 4.60 (m, 1, pyran $C_6H$), 5.23 (m, 1, naphthalene $C_8H$).

*Step D:*

Preparation of 6(R)-{2-[8'(S)(2''-ethyl-2''-methylbutyryloxy)-2'(S);6'(S)-dimethyl-1',2',3',4',4'a(S),5',6',7',8',8'a(S)-decahydronaphthyl-1'(S)-ethyl}-4(R)-hydroxy-3,4,5,6-tetrahydro-2H-pyran-2-one, $I_e$ (R'=$CH_3$)

By substituting an equimolar amount of the silyl ether $VI_e$ (R'=$CH_3$) from Example 4, Step C for the silyl ether in Step C of Example 1 and following the procedure for Step C of Example 1 there was obtained a corresponding amount of the title compound as a solid.

An analytical sample was prepared by recrystallization of the material from hexane to obtain white needles: m.p. 146—147°C.

Employing the procedure substantially as described in Example 4 Steps A through D, but substituting for the diol of structure $IV_a$ (R'=$CH_3$) in Step A, an equimolecular amount of the diol of structure $IV_a$ (R'=H) there are produced in sequence the compounds: $IV_e$ (R'=H) in Step A; $V_e$ (R'=H) in Step B; $VI_e$ (R'=H) in Step C; and $I_e$ (R'=H) in Step D.

## Example 5

6(R)-{2-[8'(S)-(2''-ethyl-2''-methylbutyryloxy)-2'(S),6'(R)-dimethyl-1',2',3',4',6',7',8'a(S)-octahydronaphthyl-1'(S)]ethyl}-4(R)-hydroxy-3,4,5,6-tetrahydro-2H-pyran-2-one, $I_b$ (R'=$CH_3$)

*Step A:*

Preparation of 6(R)-[2-(8'(S)-hydroxy-2'(S),6'(R)-dimethyl-1',2',3',4',6',7',8'a(S)-octahydronaphthyl-1'(S)-ethyl]-4(R)hydroxy-3,4,5,6-tetrahydro-2H-pyran-2-one, $IV_b$ (R'=$CH_3$)

Employing the procedure substantially as described for the preparation of the starting material $IV_a$ (R'=$CH_3$) by hydrolysis of MK-803 with refluxing aqueous $LiOH.H_2O$ for 56 hours but substituting for the MK-803 an equimolecular amount of compound $III_b$ (R'=$CH_3$) there is produced, in comparable yield, the title compound $IV_b$ (R'=$CH_3$), m.p. 136—139°C.

Following the procedure of Example 4, Steps B, C, and D, but substituting for the compound $IV_e$ (R'=$CH_3$) used in Step B thereof, an equimolecular amount of compound $IV_b$ (R'=$CH_3$) from Step A of this example, there is produced in comparable yields to those experienced in Example 4, the following compounds:

*Step B:*

6(R)-[2-(8'(S)-hydroxy-2'(S),6'(R)-dimethyl-1',2',3',4',6',7',8'a(S)-octahydronaphthyl-1'(S)-ethyl]-4(R)-(dimethyl-tert-butylsilyloxy)-3,4,5,6-tetrahydro-2H-pyran-2-one, $V_b$ (R'=$CH_3$), m.p. 140—142°C.

*Step C:*

6(R)-{2-[(8'(S)-(2''-ethyl-2''-methylbutyryloxy)-2'(S),6'(R)-dimethyl-1',2',3',4',6',7',8'a(S)-octahydronaphthyl-1'(S)]ethyl}-4(R)-(dimethyl-tert-butylsilyoxy)-3,4,5,6-tetrahydro-2H-pyran-2-one, $VI_{(b)}$ (R'=$CH_3$) wherein

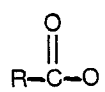 is 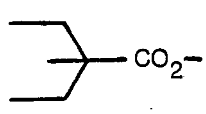

*Step D:*
6(R)-{2-[8′(S)(2″-ethyl-2″-methylbutyryloxy)-2′(S),6′(R)-dimethyl-1′,2′,3′,4′,6′,7′,8′a(S)-octahydronaphthyl-1′(S)]ethyl}-4(R)hydroxy-3,4,5,6-tetrahydro-2H-pyran-2-one, $I_b$ ($R'=CH_3$) m.p. 129—131°C. wherein

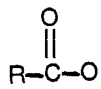 is 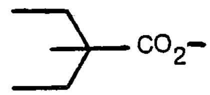

Following the procedure substantially as described in Example 5, but using $III_b$ ($R'=H$) or $III_c$, $III_d$, or $III_e$ ($R'=H$ or $CH_3$) as starting material in place of $III_b$ ($R'=CH_3$) there are produced in turn compounds $IV_b$ ($R'=H$) or $IV_{c,\,d,\,e}$ ($R'=H$ or $CH_3$), $V_b$ ($R'=H$) or $V_{c,d,e}$ ($R'=H$ or $CH_3$), $VI_b$ ($R'=H$) or $VI_{c,d,e}$ ($R'=H$ or $CH_3$), and $I_b$ ($R'=H$) or $I_{c,d,e}$ ($R'=H$ or $CH_3$) wherein

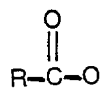 is 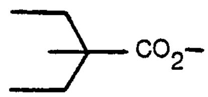

Example 6

Typical formulations for filling a size 0 hard gelatin capsule comprise 3.125, 6.25, 12.5, 25 or 50 mg of one of the novel compounds of this invention such as the products of Example 3, Step B, Example 1, Step C, or Example 2, Step B and sufficient finely divided lactose to provide a total capsule content of about 580—590 mg.

**Claims for the Contracting States: BE CH DE FR GB IT LI LU NL SE**

1. A process for the preparation of a compound of structural formula:

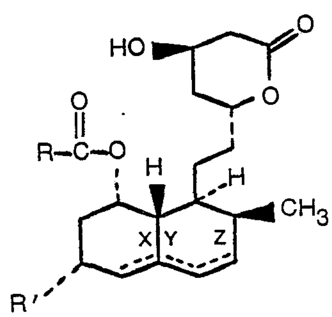

$I_{a-e}$

wherein

R′ is H or $CH_3$;

R is

(1) $C_{1-10}$ straight or branched chain alkyl except 2-butyl,

(2) $C_{3-10}$ cycloalkyl,

(3) $C_{2-10}$ alkenyl,

(4) $C_{1-10}$ $CF_3$-substituted alkyl,

(5) phenyl,

(6) halophenyl,

(7) phenyl-$C_{1-3}$ alkyl,

(8) substituted phenyl-$C_{1-3}$ alkyl in which the substituent is halo, $C_{1-3}$ alkyl or $C_{1-3}$ alkoxy;

the dotted lines at X, Y and Z represent possible double bonds, said double bonds, when any are present, being either X and Z in combination or X, Y or Z alone; and

the corresponding dihydroxy acids of the formula:

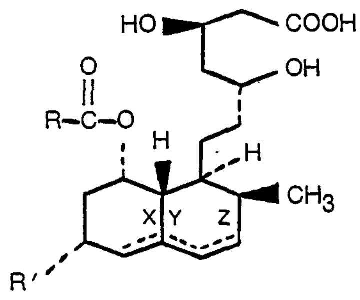

$II_{a-e}$

or a pharmaceutically acceptable salt of said acids, a $C_{1-4}$ alkyl ester of said acids or a phenyl-, dimethyl-amino-, or acetylamino-substituted-$C_{1-4}$ alkyl esters of said acids, which comprises

1) heating a compound of formula:

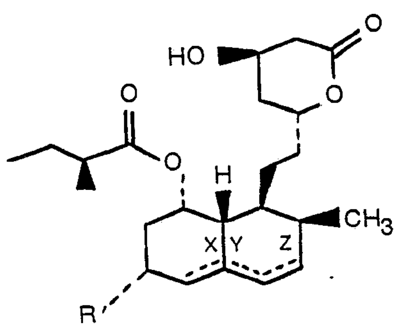

$III_{a-e}$

with an alkali metal hydroxide in a protic solvent followed by acidification and lactonization to give compound $IV_{a-e}$;

2) reacting the compound of the structure:

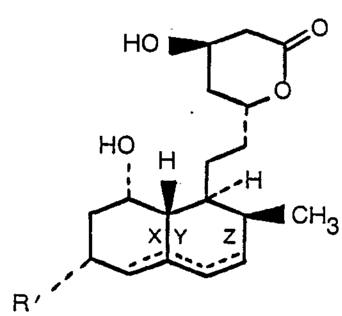

$IV_{a-e}$

with t-butyldimethylchlorosilane under an inert atmosphere at ambient temperature in the presence of an acid acceptor;

3) acylating the resulting 4-t-butyl-dimethylsilyloxy compound by:

a) stirring it in solution with an acid chloride, RCOCl, in pyridine in an inert atmosphere in the presence of an acylation catalyst, or

b) stirring it in solution at ambient temperature with an acid, RCOOH, and N,N-dicyclohexylcarbodiimide in the presence of an acylation catalyst, and

4) removing the silyl group by stirring at ambient temperature in tetrahydrofuran in the presence of 3 equivalents of tetrabutylammonium fluoride and 4 equivalents of acetic acid per equivalent of silyl compound and, if desired, treating the resulting compound $I_{a-e}$ with a base, if desired, followed by careful acidification with dilute acid, or, if desired, treating the resulting compound $I_{a-e}$ with a $C_{1-4}$-alkanol or with a phenyl-, dimethyl-amino-, or acetylamino $C_{1-4}$ alkanol.

2. The process of Claim 1 wherein R′ is $CH_3$.

3. The process of Claims 1 or 2 wherein R is $C_{3-10}$ branched alkyl except 2-butyl, especially 1-ethyl-1-methyl propyl or 1,1-diethylpropyl.

4. The process of Claims 1, 2 or 3 wherein none of X, Y or Z is a double bond.

5. The process of Claim 2 for the preparation of a compound of formula:

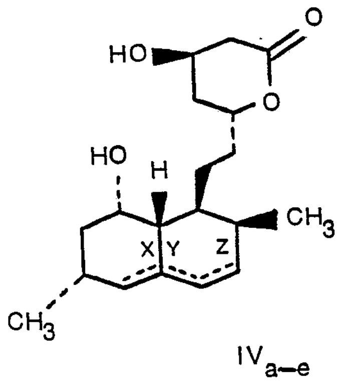

$IV_{a-e}$

or

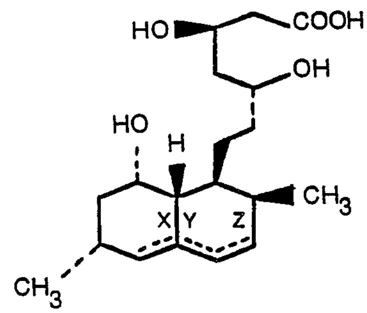

$IV'_{a-e}$

6. The process of Claim 1 for the preparation of a compound of structural formula:

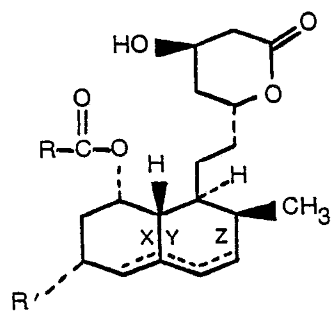

$I_{a-e}$

in which

R is

(1) $C_{1-10}$ straight or branched chain alkyl except 2-butyl,

(2) $C_{3-10}$ cycloalkyl,

(3) $C_{2-10}$ alkenyl,

(4) $C_{1-10}$ $CF_3$-substituted alkyl,

(5) phenyl,

(6) halophenyl,

(7) phenyl-$C_{1-3}$ alkyl,

(8) substituted phenyl-$C_{1-3}$ alkyl in which the substituent is halo, $C_{1-3}$ alkyl or $C_{1-3}$ alkoxy; and the dotted lines at X, Y and Z represent possible double bonds, said double bonds, when any are present, being either X and Z in combination or X, Y or Z alone; and the corresponding dihydroxy acids of the formula:

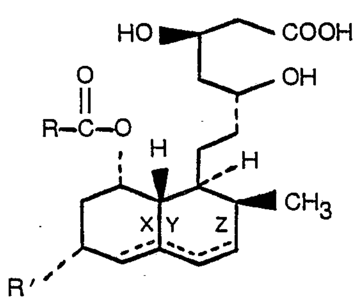

$II_{a-e}$

or a pharmaceutically acceptable salt of said acids, a $C_{1-4}$ alkyl ester of said acids or a phenyl-, dimethylamino-, or acetylamino-substituted-$C_{1-4}$ alkyl esters of said acids, which comprises

1) reacting a compound of the structure:

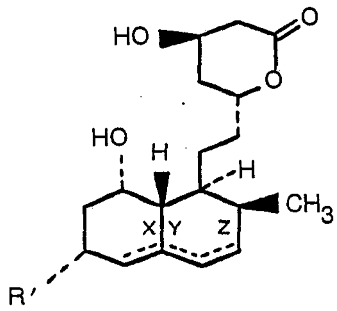

$IV_{a-e}$

with t-butyldimethylchlorosilane under an inert atmosphere at ambient temperature in the presence of an acid acceptor,

2) acylating the resulting 4-t-butyl-dimethylsilyloxy compound by:

a) stirring it in solution with an acid chloride, RCOCl, in pyridine in an inert atmosphere in the presence of an acylation catalyst, or

b) stirring it in solution at ambient temperature with an acid, RCOOH, and N,N-dicyclohexylcarbodiimide in the presence of an acylation catalyst, and

3) removing the silyl group by stirring at ambient temperature in tetrahydrofuran in the presence of 3 equivalents of tetrabutylammonium fluoride and 4 equivalents of acetic acid per equivalent of silyl compound, and, if desired, treating the resulting compound $I_{a-e}$ with a base, if desired, followed by careful acidification with dilute acid, or, if desired, treating the resulting compound $I_{a-e}$ with a $C_{1-4}$-alkanol or with a phenyl-, dimethyl-amino-, or acetylamino $C_{1-4}$ alkanol.

7. The process of Claim 6 wherein R' is $CH_3$.

8. The process of Claims 6 or 7 wherein R is $C_{3-10}$ branched alkyl except 2-butyl, especially 1-ethyl-1-methyl propyl or 1,1-diethylpropyl.

9. The process of Claims 6, 7 or 8 wherein none of X, Y or Z is a double bond.

10. A compound of the formula:

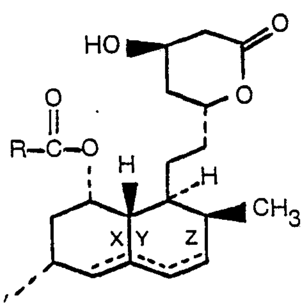

$I_{a-e}$

wherein

R' is H or $CH_3$;

R is

(1) $C_{1-10}$ straight or branched chain alkyl except 2-butyl,

(2) $C_{3-10}$ cycloalkyl,

(3) $C_{2-10}$ alkenyl,

(4) $C_{1-10}$ $CF_3$-substituted alkyl,

(5) phenyl,

(6) halophenyl,

(7) phenyl-$C_{1-3}$ alkyl,

(8) substituted phenyl-$C_{1-3}$ alkyl in which the substituent is halo, $C_{1-3}$ alkyl or $C_{1-3}$ alkoxy;

the dotted lines at X, Y and Z represent possible double bonds, said double bonds, when any are present, being either X and Z in combination or X, Y or Z alone; and

the corresponding dihydroxy acids of the formula:

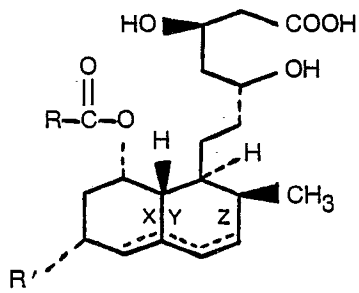

$II_{a-e}$

or a pharmaceutically acceptable salt of said acids, a $C_{1-4}$ alkyl ester of said acids or a phenyl-, dimethylamino-, or acetylamino-substituted-$C_{1-4}$ alkyl ester of said acids, with exception of compounds wherein X and Z are both double bonds and wherein

a) R'=H and

b) R'=$CH_3$ and

R=$C_{9-10}$ straight chain alkyl.

11. The compound of Claim 10 wherein R' is $CH_3$.

12. The compound of Claims 10 or 11 wherein R is $C_{3-10}$ branched chain alkyl except 2-butyl, especially is 1-ethyl-1-methylpropyl or 1,1-diethylpropyl.

13. The compound of Claims 10, 11 or 12 wherein none of X, Y or Z is a double bond.

14. A compound of the formula:

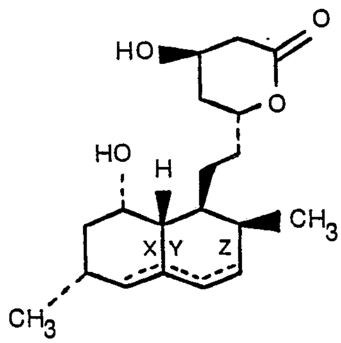

$IV_{a-e}$

or

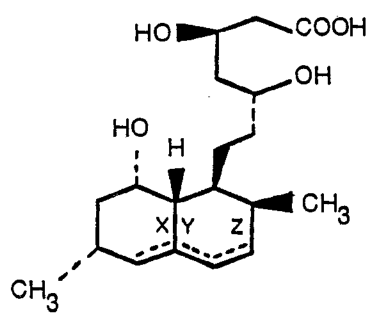

$IV'_{a-e}$

in which the dotted lines X, Y and Z represent possible double bonds, said double bonds, when any are present, being either X and Z in combination or one of X, Y or Z alone.

15. A pharmaceutical antihypercholesterolemic composition comprising a pharmaceutical carrier and an antihypercholesterolemic effective amount of a compound of any one of Claims 10 to 14.

16. A compound of the formula Ia-e according to Claims 10 or 11, wherein R is 1,1-dimethylpropyl.

17. A compound according to Claim 16, wherein none of X, Y or Z is a double bond.

18. The process of Claims 1, 2, 6 or 7, wherein R is 1,1-dimethypropyl.

19. The process of Claim 18, wherein none of X, Y and Z is a double bond.

**Claims for the Contracting State: AT**

1. A process for the preparation of a compound of structural formula:

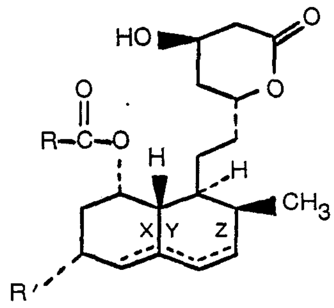

$I_{a-e}$

wherein

R' is H or $CH_3$;

R is

(1) $C_{1-10}$ straight or branched chain alkyl except 2-butyl,

(2) $C_{3-10}$ cycloalkyl,

(3) $C_{2-10}$ alkenyl,

(4) $C_{1-10}$ $CF_3$-substituted alkyl,

(5) phenyl,

(6) halophenyl,

(7) phenyl-$C_{1-3}$ alkyl,

(8) substituted phenyl-$C_{1-3}$ alkyl in which the substituent is halo, $C_{1-3}$ alkyl or $C_{1-3}$ alkoxy;

the dotted lines at X, Y and Z represent possible double bonds, said double bonds, when any are present, being either X and Z in combination or X, Y or Z alone; and

the corresponding dihydroxy acids of the formula:

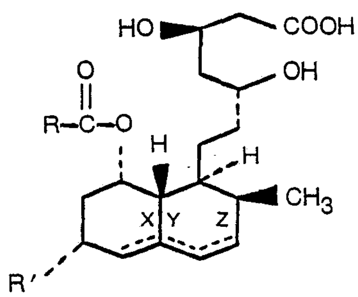

$II_{a-e}$

or a pharmaceutically acceptable salt of said acids, a $C_{1-4}$ alkyl ester of said acids or a phenyl-, dimethylamino-, or acetylamino-substituted-$C_{1-4}$ alkyl esters of said acids, which comprises

1) heating a compound of formula:

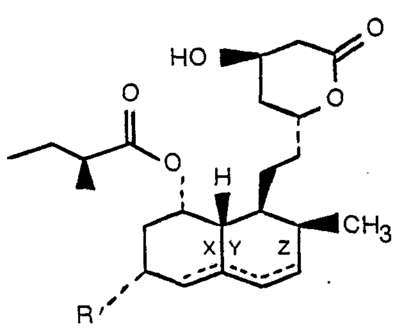

$III_{a-e}$

with an alkali metal hydroxide in a protic solvent followed by acidification and lactonization to give compound $IV_{a-e}$;

2) reacting the compound of the structure:

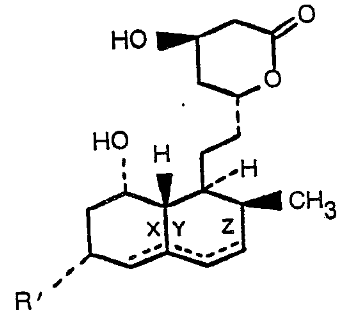

$IV_{a-e}$

with t-butyldimethylchlorosilane under an inert atmosphere at ambient temperature in the presence of an acid acceptor;

3) acylating the resulting 4-t-butyl-dimethylsilyloxy compound by:

a) stirring it in solution with an acid chloride, RCOCl, in pyridine in an inert atmosphere in the presence of an acylation catalyst, or

b) stirring it in solution at ambient temperature with an acid, RCOOH, and N,N-dicyclohexylcarbodiimide in the presence of an acylation catalyst, and

4) removing the silyl group by stirring at ambient temperature in tetrahydrofuran in the presence of 3 equivalents of tetrabutylammonium fluoride and 4 equivalents of acetic acid per equivalent of silyl compound and, if desired, treating the resulting compound $I_{a-e}$ with a base, if desired, followed by careful acidification with dilute acid, or, if desired, treating the resulting compound $I_{a-e}$ with a $C_{1-4}$-alkanol or with a phenyl-, dimethyl-amino-, or acetylamino $C_{1-4}$ alkanol.

2. The process of Claim 1 wherein R' is $CH_3$.

Priority: August 5, 1980.

3. The process of Claims 1:

wherein

R is $C_{3-10}$; and

R is

(1) $C_{1-8}$ straight chain alkyl or $C_{3-10}$ branched chain alkyl except 2-butyl,

(2) $C_{3-10}$ cycloalkyl,

(3) $C_{2-10}$ alkenyl,

(4) $C_{1-10}$ CF-substituted alkyl,

(5) phenyl,

(6) halophenyl,

(7) phenyl-$C_{1-3}$ alkyl,

(8) substituted phenyl-$C_{1-3}$ alkyl in which the substituent is halo, $C_{1-3}$ alkyl or $C_{1-3}$ alkoxy.

Priority: February 4, 1980.

4. The process of Claims 1, 2 or 3 wherein R is $C_{3-10}$ branched alkyl except 2(S)-butyl, especially 1-ethyl-1-methyl propyl or 1,1-diethylpropyl.

5. The process of Claims 1, 2, 3 or 4 wherein none of X, Y or Z is a double bond.

6. The process of Claim 2 for the preparation of a compound of formula:

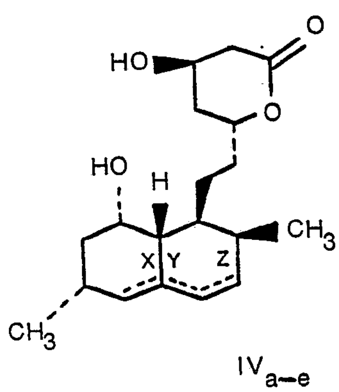

$IV_{a-e}$

or

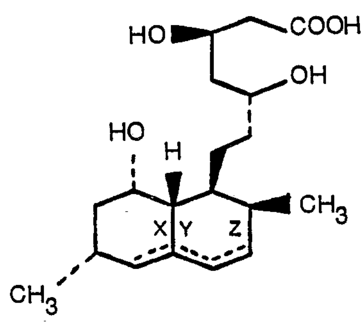

$IV'_{a-e}$

Priority: August 5, 1980

7. The process of Claim 3 for the preparation of a compound of formula:

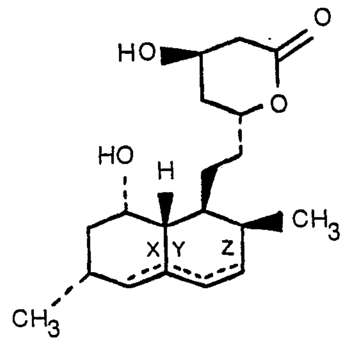

$IV_{a-e}$

or

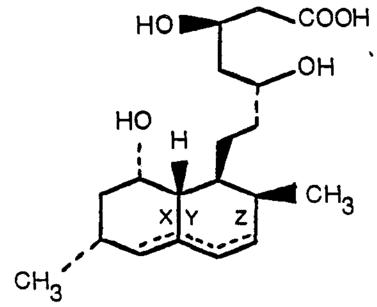

$IV'_{a-e}$

Priority: February 4, 1980

8. The process of Claim 1 for the preparation of a compound of structural formula:

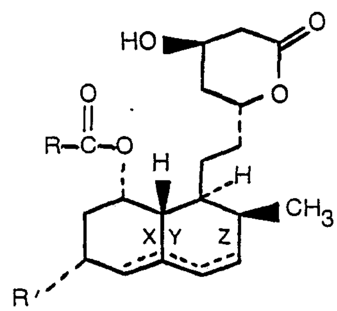

$I_{a-e}$

in which
R is
(1) $C_{1-10}$ straight or branched chain alkyl except 2-butyl,
(2) $C_{3-10}$ cycloalkyl,
(3) $C_{2-10}$ alkenyl,
(4) $C_{1-10}$ $CF_3$-substituted alkyl,
(5) phenyl,
(6) halophenyl,
(7) phenyl-$C_{1-3}$ alkyl,
(8) substituted phenyl-$C_{1-3}$ alkyl in which the substituent is halo, $C_{1-3}$ alkyl or $C_{1-3}$ alkoxy; and the dotted lines at X, Y and Z represent possible double bonds, said double bonds, when any are present, being either X and Z in combination or X, Y or Z alone; and the corresponding dihydroxy acids of the formula:

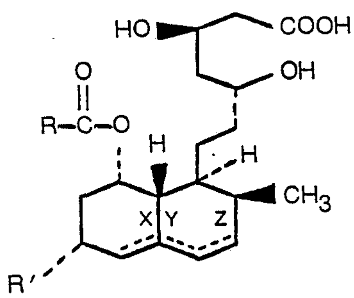

$II_{a-e}$

or a pharmaceutically acceptable salt of said acids, a $C_{1-4}$ alkyl ester of said acids or a phenyl-, dimethylamino-, or acetylamino-substituted-$C_{1-4}$ alkyl esters of said acids, which comprises

1) reacting a compound of the structure:

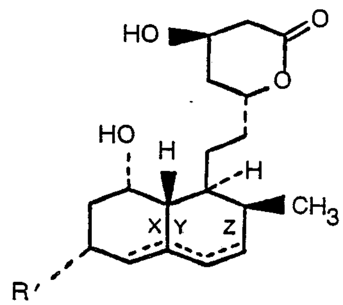

$IV_{a-e}$

with t-butyldimethylchlorosilane under an inert atmosphere at ambient temperature in the presence of an acid acceptor,

2) acylating the resulting 4-t-butyl-dimethylsilyloxy compound by:

a) stirring it in solution with an acid chloride, RCOCl, in pyridine in an inert atmosphere in the presence of an acylation catalyst, or

b) stirring it in solution at ambient temperature with an acid, RCOOH, and N,N-dicyclohexylcarbodiimide in the presence of an acylation catalyst, and

3) removing the silyl group by stirring at ambient temperature in tetrahydrofuran in the presence of 3 equivalents of tetrabutylammonium fluoride and 4 equivalents of acetic acid per equivalent of silyl compound, and, if desired, treating the resulting compound $I_{a-e}$ with a base, if desired, followed by careful acidification with dilute acid, or, if desired, treating the resulting compound $I_{a-e}$ with a $C_{1-4}$-alkanol or with a phenyl-, dimethyl-amino-, or acetylamino $C_{1-4}$ alkanol.

9. The process of Claim 8 wherein R' is $CH_3$.

Priority: August 5, 1980.

10. The process of Claim 8
wherein R' is $CH_3$; and
R is
(1) $C_{1-8}$ straight chain alkyl or $C_{3-10}$ branched chain alkyl except 2-butyl,
(2) $C_{3-10}$ cycloalkyl,
(3) $C_{2-10}$ alkenyl,
(4) $C_{1-10}$ $CF_3$-substituted alkyl,
(5) phenyl,
(6) halophenyl,
(7) phenyl-$C_{1-3}$ alkyl,
(8) substituted phenyl-$C_{1-3}$ alkyl in which the substituent is halo, $C_{1-3}$ alkyl or $C_{1-3}$ alkoxy.

Priority: February 4, 1980.

11. The process of Claims 8, 9 or 10 wherein R is $C_{3-10}$ branched alkyl except 2-butyl, especially 1-ethyl-1-methyl propyl or 1,1-diethylpropyl.

12. The process of Claims 8, 9, 10 or 11 wherein none of X, Y or Z is a double bond.

13. The process of claims 1, 2, 3, 8, 9 or 10, wherein R is 1,1-dimethylpropyl.

14. The process of Claim 13, wherein none of X, Y or Z is a double bond.

**Patentansprüche für die Vertragsstaaten: BE CH DE FR GB IT LI LU NL SE**

1. Ein Verfahren zur Herstellung einer Verbindung der Strukturformel:

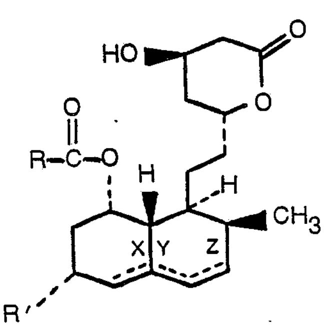

$I_{a-e}$

worin
R' H oder $CH_3$ ist;
R (1) $C_{1-10}$-geradkettiges oder verzweigtkettiges Alkyl, ausgenommen 2-Butyl,
(2) $C_{3-10}$-Cycloalkyl,
(3) $C_{2-10}$-Alkenyl,
(4) $C_{1-10}$-$CF_3$-substituiertes Alkyl,
(5) Phenyl,
(6) Halogenphenyl,
(7) Phenyl-$C_{1-3}$-alkyl oder
(8) substituiertes Phenyl-$C_{1-3}$-alkyl, in welchem der Substituent Halogen, $C_{1-3}$-Alkyl oder $C_{1-3}$-Alkoxy ist; bedeutet;

die strichlierten Linien bei X, Y und Z mögliche Doppelbindungen darstellen, wobei die genannten Doppelbindungen, wenn solche vorliegen, entweder X und Z in Kombination oder X, Y oder Z allein sind; und

der entsprecheden Dihydroxysäuren der Formel:

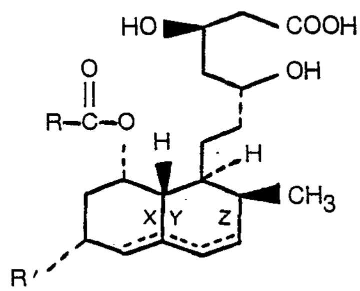

$II_{a-e}$

oder eines pharmazeutisch annehmbaren Salzes der genannten Säuren, eines $C_{1-4}$-Alkylesters der genannten Säuren oder eines phenyl-, dimethylamino- oder acetylamino-substituierten $C_{1-4}$-Alkylesters der genannten Säuren, welches umfaßt:

1) Das Erhitzen einer Verbindung der Formel:

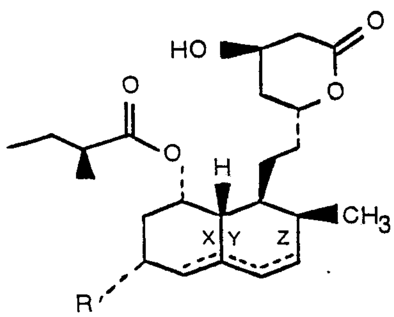

$III_{a-e}$

mit einem Alkalimetallhydroxid in einem protischen Lösungsmittel, gefolgt von der Ansäuerung und der Lactonisierung unter Bildung von Verbindung $IV_{a-e}$;

2) die Umsetzung der Verbindung der Struktur:

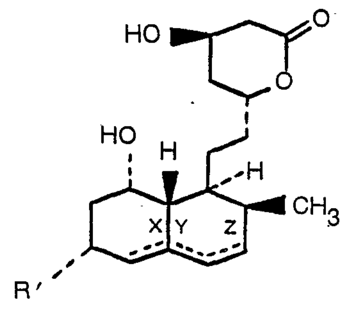

$IV_{a-e}$

mit tert.Butyldimethylchlorsilan unter einer Inertatmosphäre bei Umgebungstemperatur in der Gegenwart eines Säureakzeptors;

3) das Acylieren der entstandenen 4-tert.Butyldimethylsilyloxyverbindung durch:

a) Rühren derselben in Lösung mit einem Säurechlorid, RCOCl, in Pyridin in einer Inertatmosphäre in der Gegenwart eines Acylierungskatalysators, oder

b) Rühren derselben in Lösung bei Umgebungstemperatur mit einer Säure, RCOOH, und N,N-Dicyclohexylcarbodiimid in der Gegenwart eines Acylierungskatalysators, und

4) das Entfernen der Silylgruppe durch Rühren bei Umgebungstemperatur in Tetrahydrofuran in der Gegenwart von 3 Äquivalenten Tetrabutylammoniumfluorid und 4 Äquivalenten Essigsäure je Äquivalent Silylverbindung, und, gewünschtenfalls, die Behandlung der entstandenen Verbindung $I_{a-e}$ mit einer Base, gewünschtenfalls unter anschließender vorsichtiger Ansäuerung mit verdünnter Säure,

oder, gewünschtenfalls, die Behandlung der entstandenen Verbindung $I_{a-e}$ mit einem $C_{1-4}$-Alkanol oder mit einem Phenyl-, Dimethylamino- oder Acetylamino-$C_{1-4}$-alkanol.

2. Das Verfahren des Anspruchs 1, worin R' $CH_3$ ist.

3. Das Verfahren des Anspruchs 1 oder 2, worin R $C_{3-10}$-verzweigtes Alkyl, ausgenommen 2-Butyl, insbesondere 1-Ethyl-1-methylpropyl oder 1,1-Diethylpropyl, ist.

4. Das Verfahren des Anspruchs 1, 2 oder 3, worin keines der Symbole X, Y oder Z eine Doppelbindung bedeutet.

5. Das Verfahren des Anspruchs 2 zur Herstellung einer Verbindung der Formel:

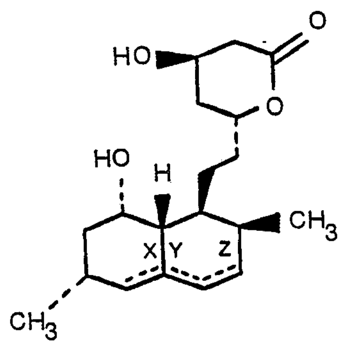

$IV_{a-e}$

oder

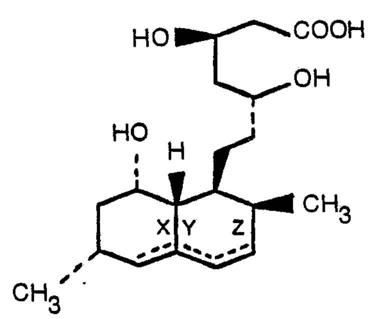

$IV'_{a-e}$

6. Das Verfahren des Anspruchs 1 zur Herstellung einer Verbindung der Strukturformel:

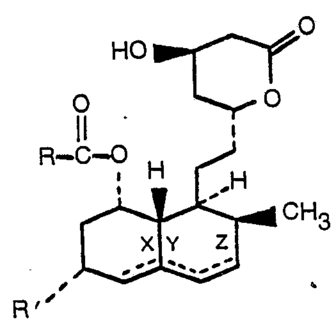

$I_{a-e}$

in welcher

R (1) $C_{1-10}$-geradkettiges oder verzweigtkettiges Alkyl, ausgenommen 2-Butyl,

(2) $C_{3-10}$-Cycloalkyl,

(3) $C_{2-10}$-Alkenyl,

(4) $C_{1-10}$-$CF_3$-substituiertes Alkyl,

(5) Phenyl,

(6) Halogenphenyl,

(7) Phenyl-$C_{1-3}$-alkyl oder

(8) substituiertes Phenyl-$C_{1-3}$-alkyl, in welchem der Substituent Halogen, $C_{1-3}$-Alkyl oder $C_{1-3}$-Alkoxy ist; bedeutet; und die strichlierten Linien bei X, Y und Z mögliche Doppelbindungen darstellen, wobei die genannten Doppelbindungen, falls vorhanden, entweder X und Z in Kombination oder X, Y oder Z allein sind; und der entsprechenden Dihydroxysäuren der Formel:

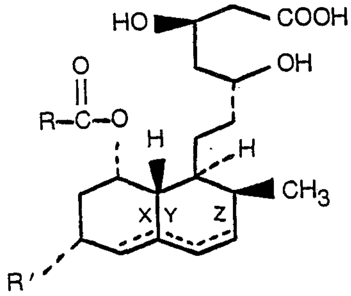

$II_{a-e}$

oder eines pharmazeutisch annehmbaren Salzes der genannten Säuren, eines $C_{1-4}$-Alkylesters der genannten Säuren oder eines phenyl-, dimethylamino- oder acetylamino-substituierten $C_{1-4}$-Alkylesters der genannten Säuren, welches umfaßt:

1) Das Umsetzen einer Verbindung der Struktur:

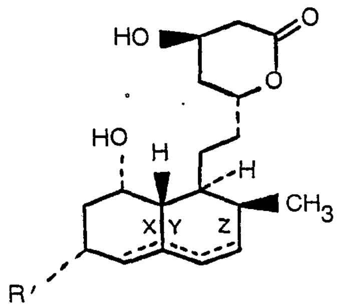

$IV_{a-e}$

mit tert.Butyldimethylchlorsilan unter einer Inertatmosphäre bei Umgebungstemperatur in der Gegenwart eines Säureakzeptors;

2) das Acylieren der entstandenen 4-tert.Butyldimethylsilyloxyverbindung durch:

a) Rühren derselben in Lösung mit einem Säurechlorid, RCOCl, in Pyridin in einer Inertatmosphäre in der Gegenwart eines Acylierungskatalysators, oder

b) Rühren derselben in Lösung bei Umgebungstemperatur mit einer Säure, RCOOH, und N,N-Dicyclohexylcarbodiimid in der Gegenwart eines Acylierungskatalysators, und

3) das Entfernen der Silylgruppe durch Rühren bei Umgebungstemperatur in Tetrahydrofuran in der Gegenwart von 3 Äquivalenten Tetrabutylammoniumfluorid und 4 Äquivalenten Essigsäure je Äquivalent Silylverbindung, und, gewünschtenfalls, die Behandlung der entstandenen Verbindung $I_{a-e}$ mit einer Base, gewünschtenfalls unter anschließender vorsichtiger Ansäuerung mit verdünnter Säure,

oder, gewünschtenfalls, die Behandlung der entstandenen Verbindung $I_{a-e}$ mit einem $C_{1-4}$-Alkanol oder mit einem Phenyl-, Dimethylamino- oder Acetylamino-$C_{1-4}$-alkanol.

7. Das Verfahren des Anspruchs 6, worin R′ $CH_3$ ist.

8. Das Verfahren des Anspruchs 6 oder 7, worin R $C_{3-10}$-verzweigtes Alkyl, ausgenommen 2-Butyl, insbesondere 1-Ethyl-1-methylpropyl oder 1,1-Diethylpropyl, ist.

9. Das Verfahren des Anspruchs 6, 7 oder 8, worin keines der Symbole X, Y oder Z eine Doppelbindung bedeutet.

10. Eine Verbindung der Formel:

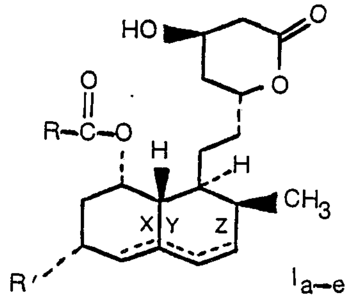

$I_{a-e}$

worin
R' H oder $CH_3$ ist;
R (1) $C_{1-10}$-geradkettiges oder verzweigtkettiges Alkyl, ausgenommen 2-Butyl,
(2) $C_{3-10}$-Cycloalkyl,
(3) $C_{2-10}$-Alkenyl,
(4) $C_{1-10}$-$CF_3$-substituiertes Alkyl,
(5) Phenyl,
(6) Halogenphenyl,
(7) Phenyl-$C_{1-3}$-alkyl oder
(8) substituiertes Phenyl-$C_{1-3}$-alkyl, in welchem der Substituent Halogen, $C_{1-3}$-Alkyl oder $C_{1-3}$-Alkoxy ist, bedeutet;

die strichlierten Linien bei X, Y und Z mögliche Doppelbindungen darstellen, wobei die genannten Doppelbindungen, wenn solche vovliegen, entweder X und Z in Kombination oder X, Y oder Z allein sind; und

die entsprechenden Dihydroxysäuren der Formel:

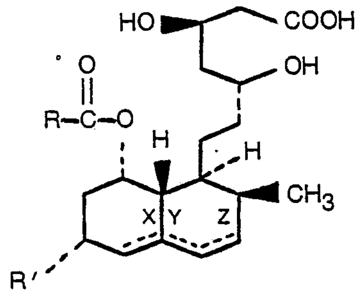

$II_{a-e}$

oder ein pharmazeutisch annehmbares Salzes der genannten Säuren, eines $C_{1-4}$-Alkylester der genannten Säuren oder ein phenyl-, dimethylamino- oder acetylamino-substituierter $C_{1-4}$-Alkylester der genannten Säuren, mit Ausnahme von Verbindungen, worin beide Symbole X und Z Doppelbindungen sind und worin
a) R' = H ist und
b) R' = $CH_3$ und
R = $C_{9-10}$-geradkettiges Alkyl sind.

11. Die Verbindung des Anspruchs 10, worin R' $CH_3$ ist.

12. Die Verbindung des Anspruchs 10 oder 11, worin R $C_{3-10}$-verzweigtkettiges Alkyl, ausgenommen 2-Butyl, insbesondere 1-Ethyl-1-methylpropyl oder 1,1-Diethylpropyl, ist.

13. Die Verbindung des Anspruchs 10, 11 oder 12, worin keines der Symbole X, Y oder Z eine Doppelbindung darstellt.

14. Eine Verbindung der Formel:

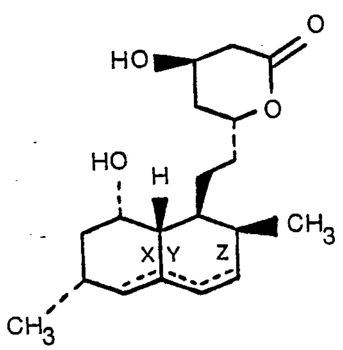

$IV_{a-e}$

oder

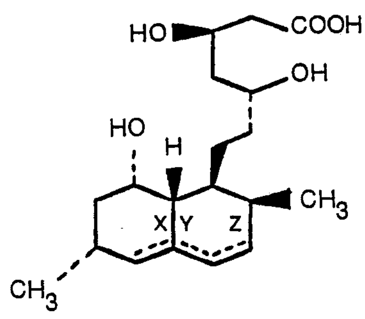

$IV'_{a-e}$

in welcher die strichlierten Linien X, Y und Z mögliche Doppelbindungen darstellen, weobei die genannten Doppelbindungen, falls vorhanden, entweder X und Z in Kombination oder eine von X, Y oder Z alleine sind.

15. Eine pharmazeutische, antihypercholesterinämische Zusammensetzung, enthaltend einen pharmazeutischen Träger und eine antihypercholesterinämisch wirksame Menge einer Verbindung nach einem der Ansprüche 10 bis 14.

16. Eine Verbindung der Formel $I_{a-e}$ nach Anspruch 10 oder 11, worin R 1,1-Dimethylpropyl ist.

17. Eine Verbindung nach Anspruch 16, worin keines der Symbole X, Y oder Z eine Doppelbindung darstellt.

18. Das Verfahren des Anspruchs 1, 2, 6 oder 7, worin R 1,1-Dimethylpropyl ist.

19. Das Verfahren des Anspruchs 18, worin keines der Symbole X, Y und Z eine Doppelbindung darstellt.

**Patentansprüche für die Vertragsstaat: AT**

1. Ein Verfahren zur Herstellung einer Verbindung der Strukturformel:

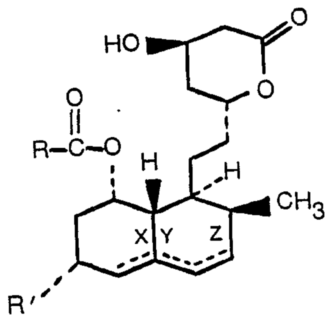

$I_{a-e}$

worin

R' H oder $CH_3$ ist;

R (1) $C_{1-10}$-geradkettiges oder verzweigtkettiges Alkyl, ausgenommen 2-Butyl,

(2) $C_{3-10}$-Cycloalkyl,

(3) $C_{2-10}$-Alkenyl,

(4) $C_{1-10}$-$CF_3$-substituiertes Alkyl,

(5) Phenyl,

(6) Halogenphenyl,

(7) Phenyl-$C_{1-3}$-alkyl oder

(8) substituiertes Phenyl-$C_{1-3}$-alkyl, in welchem der Substituent Halogen, $C_{1-3}$-Alkyl oder $C_{1-3}$-Alkoxy ist; bedeutet;

die strichlierten Linien bei X, Y und Z mögliche Doppelbindungen darstellen, wobei die genannten Doppelbindungen, wenn solche vorliegen, entweder X und Z in Kombination oder X, Y oder Z allein sind; und

der entsprecheden Dihydroxysäuren der Formel:

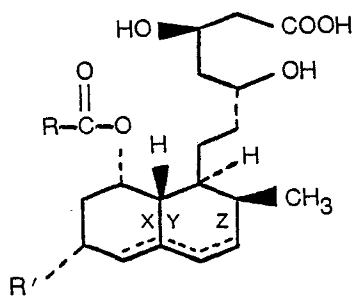

$II_{a-e}$

oder eines pharmazeutisch annehmbaren Salzes der genannten Säuren, eines $C_{1-4}$-Alkylesters der genannten Säuren oder eines phenyl-, dimethylamino- oder acetylamino-substituierten $C_{1-4}$-Alkylesters der genannten Säuren, welches umfaßt:

1) Das Erhitzen einer Verbindung der Formel:

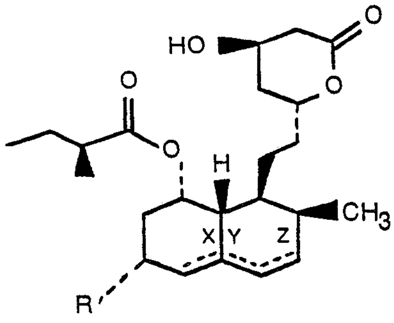

III $_{a-e}$

mit einem Alkalimetallhydroxid in einem protischen Lösungsmittel, gefolgt von der Ansäuerung und der Lactonisierung unter Bildung von Verbindung $IV_{a-e}$;

2) die Umsetzung der Verbindung der Struktur:

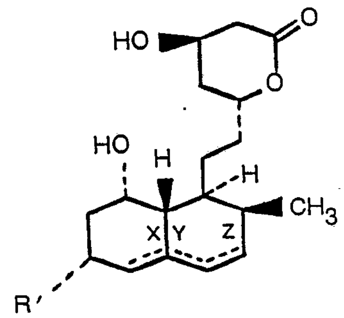

$IV_{a-e}$

mit tert.Butyldimethylchlorsilan unter einer Inertatmosphäre bei Umgebungstemperatur in der Gegenwart eines Säureakzeptors;

3) das Acylieren der entstandenen 4-tert.Butyldimethylsilyloxyverbindung durch:

a) Rühren derselben in Lösung mit einem Säurechlorid, RCOCl, in Pyridin in einer Inertatmosphäre in der Gegenwart eines Acylierungskatalysators, oder

b) Rühren derselben in Lösung bei Umgebungstemperatur mit einer Säure, RCOOH, und N,N-Dicyclohexylcarbodiimid in der Gegenwart eines Acylierungskatalysators, und

4) das Entfernen der Silylgruppe durch Rühren bei Umgebungstemperatur in Tetrahydrofuran in der Gegenwart von 3 Äquivalenten Tetrabutylammoniumfluorid und 4 Äquivalenten Essigsäure je Äquivalent Silylverbindung, und, gewünschtenfalls, die Behandlung der entstandenen Verbindung $I_{a-e}$ mit einer Base, gewünschtenfalls unter anschließender vorsichtiger Ansäuerung mit verdünnter Säure,

oder, gewünschtenfalls, die Behandlung der entstandenen Verbindung $I_{a-e}$ mit einem $C_{1-4}$-Alkanol oder mit einem Phenyl-, Dimethylamino- oder Acetylamino-$C_{1-4}$-alkanol.

2. Das Verfahren des Anspruchs 1, worin R' $CH_3$ ist.

Priorität: 5. August 1980

3. Das Verfahren des Anspruchs 1, worin R' $CH_3$ ist; und

R (1) $C_{1-8}$-geradkettiges Alkyl oder $C_{3-10}$-verzweigtkettiges Alkyl, ausgenommen 2-Butyl,
(2) $C_{3-10}$-Cycloalkyl,
(3) $C_{2-10}$-Alkenyl,
(4) $C_{1-10}$-$CF_3$-substituiertes Alkyl,
(5) Phenyl,
(6) Halogenphenyl,
(7) Phenyl-$C_{1-3}$-alkyl oder
(8) substituiertes Phenyl-$C_{1-3}$-alkyl, in welchem der Substituent Halogen, $C_{1-3}$-Alkyl oder $C_{1-3}$-Alkoxy ist; bedeutet.

Priorität: 4. Februar 1980

4. Das Verfahren des Anspruchs 1, 2 oder 3, worin R $C_{3-10}$-verzweigtes Alkyl, ausgenommen 2(S)-Butyl, insbesondere 1-Ethyl-1-methylpropy oder 1,1-Diethylpropyl, ist.

5. Das Verfahren des Anspruchs 1, 2, 3 oder 4, worin keines der Symbole X, Y oder Z eine Doppelbindung bedeutet.

6. Das Verfahren des Anspruchs 2 zur Herstellung einer Verbindung der Formel:

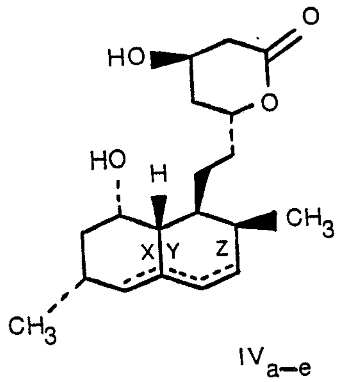

$IV_{a-e}$

oder

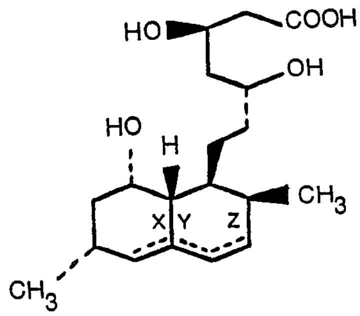

$IV'_{a-e}$

Priorität: 5. August 1980

7. Das Verfahren des Anspruchs 3 zur Herstellung einer Verbindung der Formel:

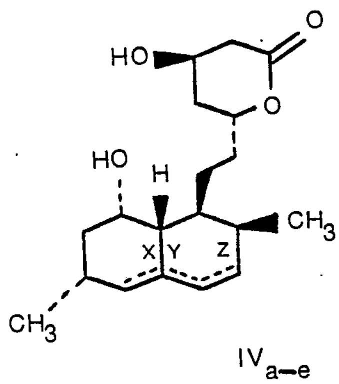

$IV_{a-e}$

oder

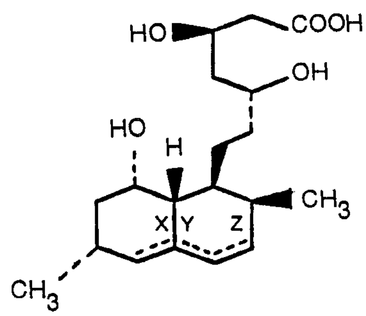

$IV'_{a-e}$

Priorität: 4. Februar 1980

8. Das Verfahren des Anspruchs 1 zur Herstellung einer Verbindung der Strukturformel:

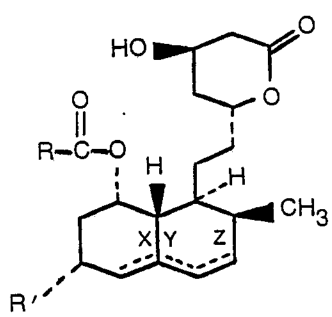

$I_{a-e}$

in welcher

R (1) $C_{1-10}$-geradkettiges oder verzweigtkettiges Alkyl, ausgenommen 2-Butyl,
(2) $C_{3-10}$-Cycloalkyl,
(3) $C_{2-10}$-Alkenyl,
(4) $C_{1-10}$-$CF_3$-substituiertes Alkyl,

(5) Phenyl,
(6) Halogenphenyl,
(7) Phenyl-$C_{1-3}$-alkyl oder
(8) substituiertes Phenyl-$C_{1-3}$-alkyl, in welchem der Substituent Halogen, $C_{1-3}$-Alkyl oder $C_{1-3}$-Alkoxy ist; bedeutet; und die strichlierten Linien bei X, Y und Z mögliche Doppelbindungen darstellen, wobei die genannten Doppelbindungen, falls vorhanden, entweder X und Z in Kombination oder X, Y oder Z allein sind; und der entsprechenden Dihydroxysäuren der Formel:

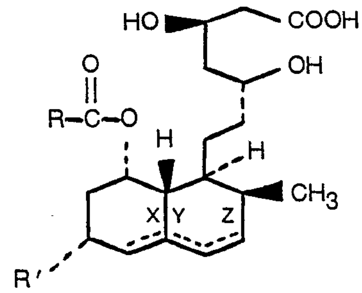

$II_{a-e}$

oder eines pharmazeutisch annehmbaren Salzes der genannten Säuren, eines $C_{1-4}$-Alkylesters der genannten Säuren oder eines phenyl-, dimethylamino- oder acetylamino-substituierten $C_{1-4}$-Alkylesters der genannten Säuren, welches umfaßt:

1) Das Umsetzen einer Verbindung der Struktur:

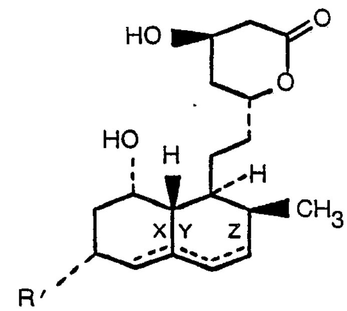

$IV_{a-e}$

mit tert.Butyldimethylchlorsilan unter einer Inertatmosphäre bei Umgebungstemperatur in der Gegenwart eines Säureakzeptors;

2) das Acylieren der entstandenen 4-tert.Butyldimethylsilyloxyverbindung durch:

a) Rühren derselben in Lösung mit einem Säurechlorid, RCOCl, in Pyridin in einer Inertatmosphäre in der Gegenwart eines Acylierungskatalysators, oder

b) Rühren derselben in Lösung bei Umgebungstemperatur mit einer Säure, RCOOH, und N,N-Dicyclohexylcarbodiimid in der Gegenwart eines Acylierungskatalysators, und

3) das Entfernen der Silylgruppe durch Rühren bei Umgebungstemperatur in Tetrahydrofuran in der Gegenwart von 3 Äquivalenten Tetrabutylammoniumfluorid und 4 Äquivalenten Essigsäure je Äquivalent Silylverbindung, und, gewünschtenfalls, die Behandlung der entstandenen Verbindung $I_{a-e}$ mit einer Base, gewünschtenfalls unter anschließender vorsichtiger Ansäuerung mit verdünnter Säure,

oder, gewünschtenfalls, die Behandlung der entstandenen Verbindung $I_{a-e}$ mit einem $C_{1-4}$-Alkanol oder mit einem Phenyl-, Dimethylamino- oder Acetylamino-$C_{1-4}$-alkanol.

9. Das Verfahren des Anspruchs 6, worin R′ $CH_3$ ist.

Priorität: 5 August 1980

10. Das Verfahren des Anspruchs 8, worin R′ $CH_3$ ist; und

R (1) $C_{1-8}$-geradkettiges Alkyl oder $C_{3-10}$-verzweigtkettiges Alkyl, ausgenommen 2-Butyl,
(2) $C_{3-10}$-Cycloalkyl,
(3) $C_{2-10}$-Alkenyl,
(4) $C_{1-10}$-$CF_3$-substituiertes Alkyl,
(5) Phenyl,
(6) Halogenphenyl,
(7) Phenyl-$C_{1-3}$-alkyl oder

(8) substituiertes Phenyl-$C_{1-3}$-alkyl, in welchem der Substituent Halogen, $C_{1-3}$-Alkyl oder $C_{1-3}$-Alkoxy ist, bedeutet.

Priorität: 4. Februar 1980

11. Das Verfahren des Anspruchs 8, 9 oder 10, worin R $C_{3-10}$-verzweigtes Alkyl, ausgenommen 2-Butyl, insbesondere 1-Ethyl-1-methylpropyl oder 1,1-Diethylpropyl, ist.

12. Das Verfahren des Anspruchs 8, 9, 10 oder 11, worin keines der Symbole X, Y oder Z eine Doppelbindung bedeutet.

13. Das Verfahren des Anspruchs 1, 2, 3, 8, 9 oder 10, worin R 1,1-Dimethylpropyl ist.

14. Das Verfahren des Anspruchs 13, worin keines der Symbole X, Y oder Z eine Doppelbindung darstellt.

**Revendications pour les Etats contractants: BE CH DE FR GB IT LI LU NL SE**

1. Un procédé pour la préparation d'un composé de formule développée:

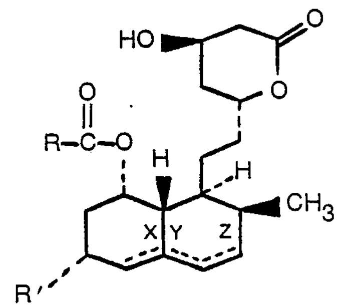

$I_{a-e}$

dans laquelle

R' est H ou $CH_3$;

R est

(1) un alkyle en $C_{1-10}$ à chaîne droite ou ramifiée, à l'exception de 2-butyle,

(2) un cycloalkyle en $C_{3-10}$,

(3) un alcényle en $C_{2-10}$,

(4) un alkyle en $C_{1-10}$ substitué par $-CF_3$,

(5) un phényle,

(6) un halogénophényle,

(7) un phényl-alkyle en $C_{1-3}$,

(8) un phényl-alkyle en $C_{1-3}$ substitué dont le substituant est un halogéno, un alkyle en $C_{1-3}$ ou un alcoxy en $C_{1-3}$;

Les pointillés en X, Y et Z représentent des doubles liaisons éventuelles, lesdites doubles liaisons, lorsqu'elles sont présentes, étant soit X et Z en combinaison, soit X, Y ou Z seuls; et

Les dihydroxy-acides correspondants de formule:

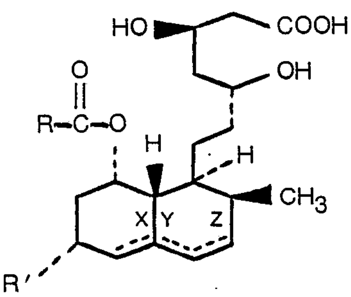

$II_{a-e}$

ou un sel acceptable en pharmacie desdits acides, un ester d'alkyle en $C_{1-4}$ desdits acides ou un ester d'alkyle en $C_{1-4}$ substitué par les groupes phényles diméthylamino ou acétylamino desdits acides, qui comprend

1) le chauffage d'un composé de formule:

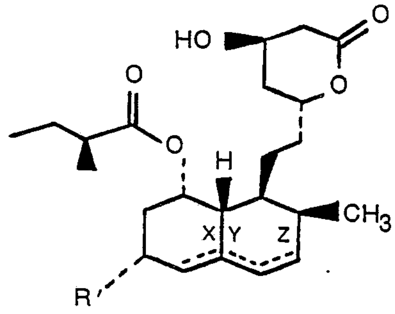

$III_{a-e}$

avec un hydroxyde de métal alcalin dans un solvant protique suivi d'une acidification et d'une lactonisation pour fournir un composé $IV_{a-e}$;

2) la réaction du composé de structure:

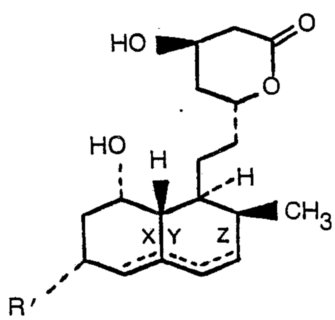

$IV_{a-e}$

avec le tert-butyldiméthylchhorosilane dans une atmosphère inerte à la température ambiante en présence d'un accepteur d'acide;

3) l'acylation du composé de type 4-tert-butyldiméthylsilyloxy obtenu par:

a) agitation de celui-ci en solution avec un chlorure d'acide, RCOCl, dans la pyridine dans une atmosphère inerte en présence d'un catalyseur d'acylation, ou

b) agitation de celui-ci en solution à la température ambiante avec un acide, RCOOH, et le N,N-dicyclohexylcarbodiimide en présence d'un catalyseur d'acylation, et

4) l'élimination du groupe silyle par agitation à la température ambiante dans le tétrahydrofuranne en présence de trois équivalents de fluorure de tétrabutylammonium et de quatre équivalents d'acide acétique par équivalent de composé silylique, et, si on le désire, traitement du composé $I_{a-e}$ obtenu avec une base, si on le désire, suivi d'une acidification ménagée avec un acide dilué ou, si on le désire, traitement du composé obtenu $I_{a-e}$ avec un alcanol en $C_{1-4}$ ou avec un phényl-, diméthylamino- ou acétylamino-alcanol en $C_{1-4}$.

2. Le procédé de la revendication 1, où R' est $CH_3$.

3. Le procédé de la revendication 1 ou 2, où R est un alkyle en $C_{3-10}$, à l'exception de 2-butyle, en particulier un 1-éthyl-1-méthylpropyle ou un 1,1-diéthylpropyle.

4. Le procédé de la revendication 1, 2 ou 3, où aucun de X, Y ou Z n'est une double liaison.

5. Le procédé de la revendication 2 pour la préparation d'un composé de formule:

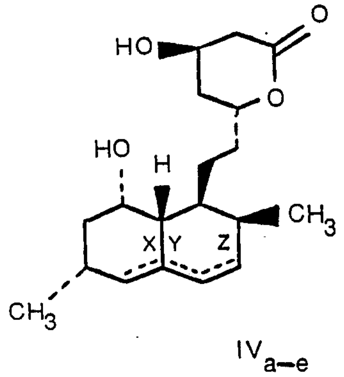

$IV_{a-e}$

ou

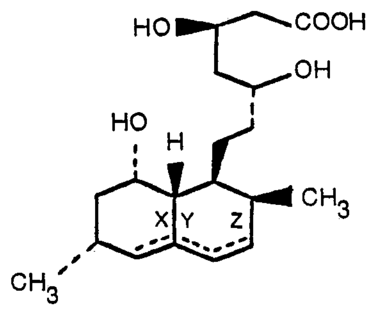

$IV'_{a-e}$

6. Le procédé de la revendication 1 pour la préparation d'un composé de formule développée:

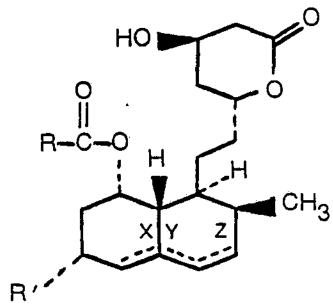

$I_{a-e}$

dans laquelle R' est

(1) un alkyle chaîne droite ou ramifiée en $C_{1-10}$, à l'exception de 2-butyle,
(2) un cycloalkyle en $C_{3-10}$,
(3) un alcényle en $C_{2-10}$,
(4) un alkyle en $C_{1-10}$ substitué par $-CF_3$,
(5) un phényle,
(6) un halogénophényle,
(7) un phényl-alkyle en $C_{1-3}$,
(8) un phényl-alkyle en $C_{1-3}$ substitué dont le substituant est un halogéno, un alkyle en $C_{1-3}$ ou un alcoxy en $C_{1-3}$; et les pointillés en X, Y et Z représentent des doubles liaisons éventuelles, lesdites doubles liaisons, lorsqu'elles sont présentes, étant soit X et Z en combinaison, soit X, Y ou Z seuls; et les dihydroxy-acides correspondants de formule:

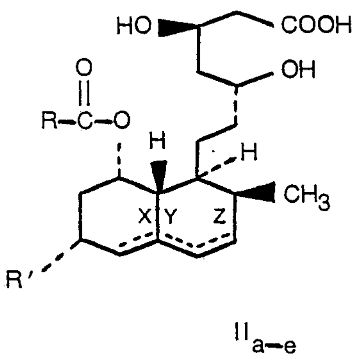

$II_{a-e}$

ou un sel acceptable en pharmacie desdits acides, un ester d'alkyle en $C_{1-4}$ desdits acides ou un ester d'alkyle en $C_{1-4}$ substitué par les groupes phényles diméthylamino ou acétylamino desdits acides, qui comprend

1) la réaction d'un composé de structure:

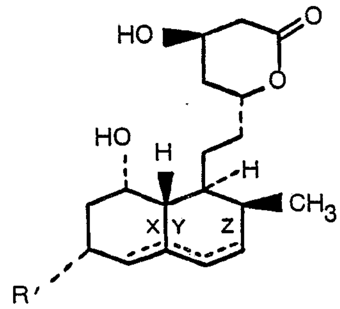

$IV_{a-e}$

avec le tert-butyldiméthylchlorosilane dans une atmosphère inerte à la température ambiante en présence d'un accepteur d'acide,

2) l'acylation du composé de type 4-tert-butyldiméthylsilyloxy obtenu par:

a) agitation de celui-ci en solution avec un chlorure d'acide, RCOCl, dans la pyridine dans une atmosphère inerte en présence d'un catalyseur d'acylation, ou

b) agitation de celui-ci en solution à la température ambiante avec un acide, RCOOH, et le N,N-dicyclohexylcarbodiimide en présence d'un catalyseur d'acylation, et

3) l'élimination du groupe silyle par agitation à la température ambiante dans le tétrahydrofuranne en présence de trois équivalents de fluorure de tétrabutylammonium et de quatre équivalents d'acide acétique par équivalent de composé silylique, et, si on le désire, le traitement du composé obtenu $I_{a-e}$ avec une base, si on le désire, suivi d'une acidification ménagée avec un acide dilué ou, si on le désire, traitement du composé obtenu $I_{a-e}$ avec un alcanol en $C_{1-4}$ ou avec un phényl-, diméthylamino- ou acétylamino-alcanol en $C_{1-4}$.

7. Le procédé de la revendication 6, où R' est $CH_3$.

8. Le procédé des revendications 6 ou 7, où R est un alkyle ramifié en $C_{3-10}$, à l'exception de 2-butyle, en particulier 1-éthyl-1-méthylpropyle ou 1,1-diéthylpropyle.

9. Le procédé des revendications 6, 7 ou 8, où aucun de X, Y ou Z n'est une double liaison.

10. Un composé de formule:

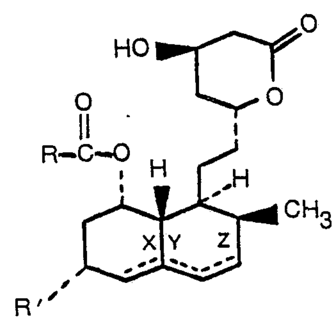

$I_{a-e}$

dans laquelle

R' est H ou $CH_3$;

R est

(1) un alkyle à chaîne droite ou ramifiée en $C_{1-10}$, à l'exception de 2-butyle,

(2) un cycloalkyle en $C_{3-10}$,

(3) un alcényle en $C_{2-10}$,

(4) un alkyle en $C_{1-10}$ substitué dont le substituant est un halogéno, un alkyle en $C_{1-3}$ ou un alcoxy en $C_{1-3}$;

les pointillés en X, Y et Z représentent des doubles liaisons éventuelles, lesdites doubles liaisons, lorsqu'elles sont présentes, étant soit X et Y en combinaison, soit X, Y ou Z seuls; et

les dihydroxy-acides correspondants de formule:

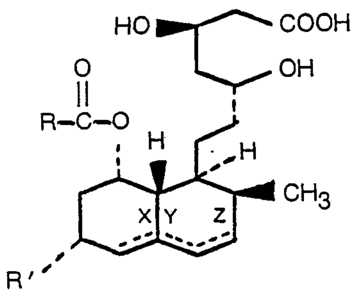

$II_{a-e}$

ou un sel acceptable en pharmacie desdits acides, un ester d'alkyle en $C_{1-4}$ desdits acides ou un ester d'alkyle en $C_{1-4}$ substitué par un groupe phényle, diméthylamino ou acétylamino desdits acides, à l'exception des composés où X et Y sont tous deux des doubles liaisons et où

a) R' = H et

b) R' = $CH_3$, et

R = un alkyle à chaîne droite en $C_{9-10}$.

11. Le composé de la revendication 10, où R' est $CH_3$.

12. Le composé des revendications 10 où 11, où R est un alkyle à chaîne ramifiée en $C_{3-10}$, à l'exception de 2-butyle, en particulier est un l-éthyl-1-méthylpropyle ou un 1,1-diéthylpropyle.

13. Le composé de la revendication 10, 11 ou 12, où aucun de X, Y ou Z n'est une double liaison.

14. Un composé de formule:

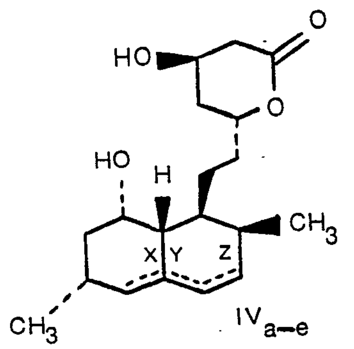

$IV_{a-e}$

ou

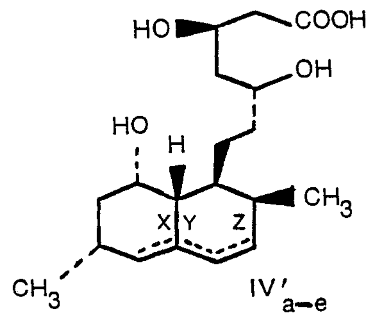

$IV'_{a-e}$

où les pointillés X, Y et Z représentent des doubles liaisons éventuelles, les doubles liaisons, lorsqu'elles sont présentes, étant soit X et Y en combinason, soit un de X, Y ou Z seul.

15. Une composition pharmaceutique antihypercholestérolémiante, comprenant un support pharmaceutique et une quantité antihypercholestérolémiante efficace d'un composé de l'une quelconque des revendications 10 à 14.

16. Un composé de formule $I_{a-e}$ selon les revendications 10 ou 11, où R est un 1,1-diméthylpropyle.

17. Un composé selon la revendication 16, où aucun de X, Y ou Z n'est une double liaison.

18. Le procédé des revendications 1, 2, 6 ou 7 où R est un 1,1-diméthylpropyle.

19. Le procédé de la revendication 18, où aucun de X, Y et Z n'est une double liaison.

**Revendications pour les Etat contractant: AT**

1. Un procédé pour la préparation d'un composé de formule développée:

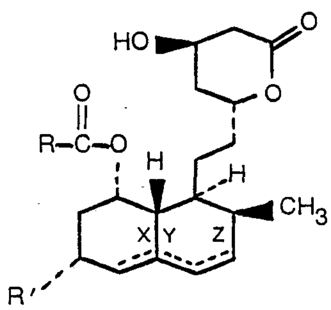

$I_{a-e}$.

dans laquelle

R' est H ou $CH_3$;

R est

(1) un alkyle en $C_{1-10}$ à chaîne droite ou ramifiée, à l'exception de 2-butyle,

(2) un cycloalkyle en $C_{3-10}$,

(3) un alcényle en $C_{2-10}$,

(4) un alkyle en $C_{1-10}$ substitué par $-CF_3$,

(5) un phényle,

(6) un halogénophényle,

(7) un phényl-alkyle en $C_{1-3}$,

(8) un phényl-alkyle en $C_{1-3}$ substitué dont le substituant est un halogéno, un alkyle en $C_{1-3}$ ou un alcoxy en $C_{1-3}$;

Les pointillés en X, Y et Z représentent des doubles liaisons éventuelles, lesdites doubles liaisons, lorsqu'elles sont présentes, étant soit X et Z en combinaison, soit X, Y ou Z seuls; et

Les dihydroxy-acides correspondants de formule:

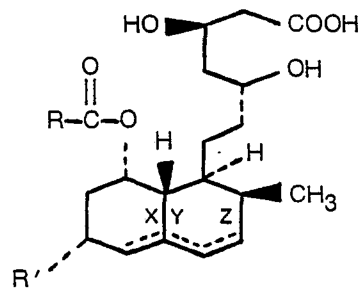

$II_{a-e}$

ou un sel acceptable en pharmacie desdits acides, un ester d'alkyle en $C_{1-4}$ desdis acides ou un ester d'alkyle en $C_{1-4}$ substitué par les groupes phényles diméthylamino ou acétylamino desdits acides, qui comprend

1) le chauffage d'un composé de formule:

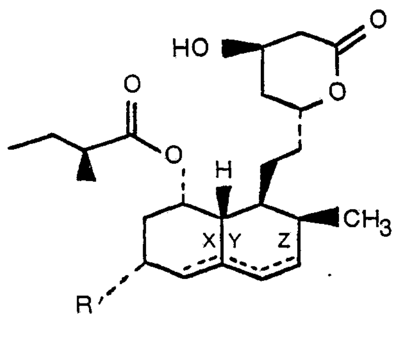

$III_{a-e}$

avec un hydroxyde de métal alcalin dans un solvant protique suivi d'une acidification et d'une lactonisation pour fournir un composé $IV_{a-e}$;

2) la réaction du composé de structure:

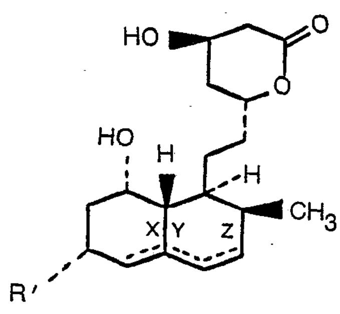

$IV_{a-e}$

avec le tert-butyldiméthychlorosilane dans une atmosphère inerte à la température ambiante en présence d'un accepteur d'acide;

3) l'acylation du composé de type 4-tert-butyldiméthylsilyloxy obtenu, par:

a) agitation de celui-ci en solution avec un chlorure d'acide, RCOCl, dans la pyridine dans une atmosphère inerte en présence d'un catalyseur d'acylation, ou

b) agitation de celui-ci en solution à la température ambiante avec un acide, RCOOH, et le N,N-dicyclohexylcarbodiimide en présence d'un catalyseur d'acylation, et

4) l'élimination du groupe silyle par agitation à la température ambiante dans le tétrahydrofuranne en présence de trois équivalents de fluorure de tétrabutylammonium et de quatre équivalents d'acide acétique par équivalent de composé silylique, et, si on le désire, traitement du composé $I_{a-e}$ obtenu avec une base, si on le désire, suivi d'une acidification ménagée avec un acide dilué ou, si on le désire, traitement du composé obtenu $I_{a-e}$ avec un alcanol en $C_{1-4}$ ou avec un phényl-, diméthylamino- ou acétylamino-alcanol en $C_{1-4}$.

2. Le procédé de la revendication 1, où Ŕ est $CH_3$.

3. Le procédé de la revendication 1:
ou R' est $CH_3$; et
R est
(1) un alkyle à chaîne droite en $C_{1-8}$ ou un alkyle à chaîne ramifiée en $C_{3-10}$, à l'exception de 2-butyle,
(2) un cycloalkyle en $C_{3-10}$,
(3) un alcényle en $C_{2-10}$,
(4) un alkyle en $C_{1-10}$ substitué par $—CF_3$,
(5) un phényle,
(6) un halogénophényle,
(7) un phényl-alkyle en $C_{1-3}$,
(8) un phényl-alkyle en $C_{1-3}$ substitué dont le substituant est un halogéno, un alkyle en $C_{1-3}$ ou un alcoxy en $C_{1-3}$.

4. Le procédé de la revendications 1, 2 ou 3, où R est un alkyle ramifié en $C_{3-10}$, à l'exception de 2(S)-butyle, en particulier un l-éthyl-1-méthylpropyle ou un 1,1-diéthylpropyle.

5. Le procédé des revendications 1, 2, 3 ou 4, où aucun de X, Y ou Z n'est une double liaison.

6. Le procédé de la revendication 2 pour la préparation d'un composé de formule:

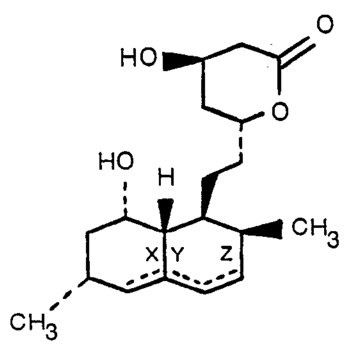

$IV_{a-e}$

ou

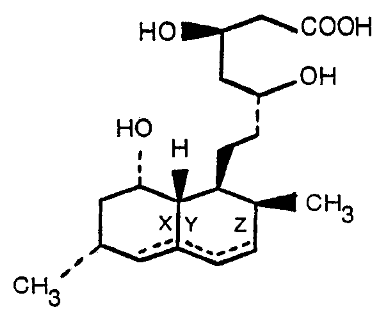

$IV'_{a-e}$

7. Le procédé de la revendication 3 pour la préparation d'un composé de formule:

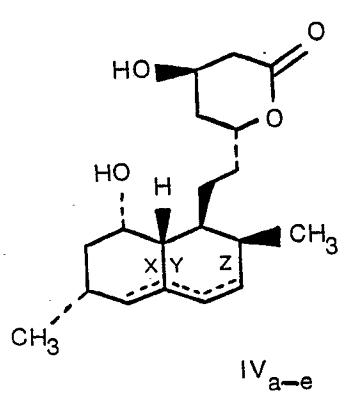

$IV_{a-e}$

ou

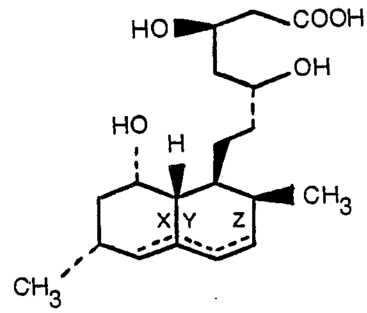

$IV'_{a-e}$

8. Le procédé de la revendication 1 pour la préparation d'un composé de formule développée:

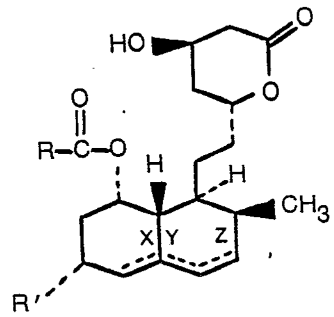

$I_{a-e}$

dans laquelle R est

(1) un alkyle a chaîne droite ou ramifiée en $C_{1-10}$, à l'exception de 2-butyle,

(2) un cycloalkyle en $C_{3-10}$,

(3) un alcényle en $C_{2-10}$,

(4) un alkyle en $C_{1-10}$ substitué par $—CF_3$,

(5) un phényle,

(6) un halogénophényle,

(7) un phényl-alkyle en $C_{1-3}$,

(8) un phényl-alkyle en $C_{1-3}$ substitué dont le substituant est un halogéno, un alkyle en $C_{1-3}$ ou un alcoxy en $C_{1-3}$; et les pointillés en X, Y et Z représentent des doubles liaisons éventuelles, lesdites doubles liaisons, lorsqu'elles sont présentes, étant soit X et Z en combinaison, soit X, Y ou Z seuls; et les dihydroxy-acides correspondants de formule:

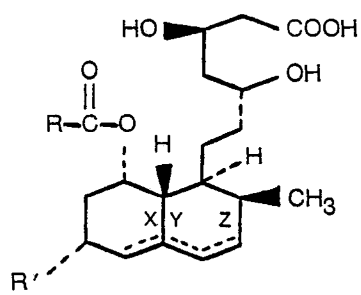

$II_{a-e}$

ou un sel acceptable en pharmacie desdits acides, un ester d'alkyle en $C_{1-4}$ desdits acides ou un ester d'alkyle en $C_{1-4}$ substitué par des groupes phényles diméthylamino ou acétylamino desdits acides, qui comprend

1) le réaction d'un composé de structure:

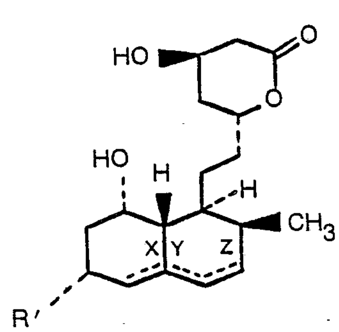

$IV_{a-e}$

avec le tert-butyldiméthylchlorosilane dans une atmosphère inerte à la température ambiante en présence d'un accepteur d'acide,

2) l'acylation du composé de type 4-tert-butyldiméthylsilyloxy obtenu par:

a) agitation de celui-ci en solution avec un chlorure d'acide, RCOCl, dans la pyridine dans une atmosphère inerte en présence d'un catalyseur d'acylation, ou

b) l'agitation de celui-ci en solution à la température ambiante avec un acide, RCOOH, et le N,N-dicyclohexylcarbodiimide en présence d'un catalyseur d'acylation, et

3) l'élimination du groupe silyle par agitation à la température ambiante dans le tétrahydrofuranne en présence de trois équivalents de fluorure de tétrabutylammonium et de quatre équivalents d'acide acétique par équivalent de composé silylique, et, si on le désire, traitement du composé $I_{a-e}$ obtenu avec une base, si on le désire, suivi d'une acidification ménagée avec un acide dilué ou, si on le désire, traitement du composé obtenu $I_{a-e}$ avec un alcanol en $C_{1-4}$ ou avec un phényl-, diméthylamino- ou acétylaminoalcanol en $C_{1-4}$.

9. Le procédé de la revendication 8, où R' est $CH_3$.

10. Le procédé de la revendication 8, où R' est $CH_3$; et R et (1) un alkyle a chaîne droite en $C_{1-8}$ ou un alkyle à chaîne ramifiée en $C_{3-10}$, à l'exception de 2-butyle,

(2) un cycloalkyle en $C_{3-10}$,

(3) un alcényle en $C_{2-10}$,

(4) un alkyle en $C_{1-10}$ substitué par $-CF_3$,

(5) un phényle,

(6) un halogénophényle,

(7) un phényl-alkyle en $C_{1-3}$,

(8) un phényl-alkyle en $C_{1-3}$ substitué dont le substituant est un halogéno, un alkyle en $C_{1-3}$ ou un alcoxy en $C_{1-3}$.

11. Le Procédé des revendications 8, 9 ou 10, où R est un alkyle ramifié en $C_{3-10}$, à l'exception de 2-butyle, en particulier un l-éthyl-1-méthylpropyle ou un 1,1-diéthylpropyle.

12. Le procédé des revendications 8, 9, 10 ou 11, où aucun de X, Y ou Z n'est une double liaison.

13. Le procédé des revendications 1, 2, 3, 8, 9 ou 10, où R est un 1,1-diméthylpropyle.

14. Le procédé de la revendication 13, où aucun de X, Y ou Z n'est une double liaison.